# EUROPEAN PATENT SPECIFICATION

(11) **EP 1 226 279 B1**
(45) Date of publication and mention of the grant of the patent: **29.03.2006**
(21) Application number: 00975512.5
(22) Date of filing: 30.10.2000
(51) Int. Cl.: C12Q 1/68, G01N 33/53, A61K 39/395, C12N 15/11, C07H 21/04, C07K 14/47

(54) **MODULATION OF NEUROENDOCRINE DIFFERENTIATION BY PROTEIN 25.1**
MODULATION DER NEUROKRINEN DIFFERENZIERUNG DURCH PROTEIN 25.1
MODULATION DE LA DIFFERENCIATION NEUROENDOCRINIENNE PAR LA PROTEINE 25.1

(30) Priority: 29.10.1999 US 162393 P
(43) Date of publication of application: 31.07.2002
(73) Proprietor: OREGON HEALTH SCIENCES UNIVERSITY, Portland, OR 97201 (US)
(72) Inventor: WILSON, Elizabeth, Tualatin, OR 97062 (US); ROSENFELD, Ron, G., Tualatin, OR 97062 (US); OH, Youngman, Beaverton, OR 97006 (US)
(74) Representative: Wittop Koning, Tom Hugo
(86) International application number: PCT/US2000/029975
(87) International publication number: WO 2001/032925

(56) References cited:
- WO-A-98/45427
- LAI CHUN-HUNG ET AL: "Identification of novel human genes evolutionarily conserved in Caenorhabditis elegans by comparative proteomics." GENOME RESEARCH, vol. 10, no. 5, May 2000 (2000-05), pages 703-713, XP000929862 ISSN: 1088-9051 & DATABASE NCBI [Online] 1 June 1999 (1999-06-01) LAI: "Identification of Novel Human Genes Evolutionarily Conserved genes" retrieved from EBI Database accession no. AF151907
- WILSON ELIZABETH M ET AL: "Interaction of IGF-binding protein-related protein 1 with a novel protein, neuroendocrine differentiation factor, results in neuroendocrine differentiation of prostate cancer cells." JOURNAL OF CLINICAL ENDOCRINOLOGY & METABOLISM, vol. 86, no. 9, September 2001 (2001-09), pages 4504-4511, XP002235156 ISSN: 0021-972X & DATABASE SWISSPROT [Online] 1 October 2000 (2000-10-01) WILSON: "Neuroendocrine differentiation factor" retrieved from EBI Database accession no. Q9NZ51
- VORWERK ET AL.: 'CTGF (IGFBP-rP2) is specially expressed in malignant lymphoblasts of patients with acute lymphoblastic leukaemia (ALL)' BRITISH JOURNAL OF CANCER vol. 83, no. 6, 2000, pages 756 - 760, XP002939645
- DEGEORGES ET AL.: 'Distribution of IGFBP-rP1 in normal human tissues' THE JOURNAL OF HISTOCHEMISTRY AND CYTOCHEMISTRY vol. 48, no. 6, 2000, pages 747 - 754, XP002939647
- DEGEORGES ET AL.: 'Human prostate cancer expresses the low affinity insulin-like growth factor binding protein IGFBP-rP1' CANCER RESEARCH vol. 59, 15 June 1999, pages 2787 - 2790, XP002939646
- SPRENGER ET AL.: 'Insulin-like growth factor binding protein-related protein 1 (IGFBP-rP1) is a potential tumor suppressor protein for prostate cancer' CANCER RESEARCH vol. 59, 15 May 1999, pages 2370 - 2375, XP002939648
- KIAM ET AL.: 'Insulin-like growth factor binding proteins (IGFBPs) and IGFBP-related protein 1-levels in cerebrospinal fluid of children with acute lymphoblastic leukemia' JOURNAL OF CLINICAL ENDOCRINOLOGY AND METABOLISM vol. 84, no. 4, 1999, pages 1283 - 1287, XP002939649
- HAUGK ET AL.: 'Insulin-like growth factor (IGF)-binding protein-related protein-1: An autocrine/paracrine factor that inhibits skeletal myoblast differentiation but permits proliferation in response to IGF' ENDOCRINOLOGY vol. 141, no. 1, 2000, pages 100 - 110, XP002939650

## Description

### TECHNICAL FIELD OF THE INVENTION

The present invention provides drug candidate screening assays, diagnostic assays, and therapeutic methods for tumor suppression and the treatment of cancer, including preventing induction of neuroendocrine differentiation in cancer cells, utilizing a novel IGFBP-rP1 interacting protein called P25.1 as the intervention target. The present invention further provides a P25.1 cDNA sequence, a P25.1 polypeptide and fragments thereof, an anti-P25.1 antibody, and anti-25.1 antisense oligonucleotides.

### BACKGROUND OF THE INVENTION

### Neuroendocrine Differentianon Correlates with Cancer Progression.

Neuroendocrine ("NE") cells have been implicated in many cancers including, small cell lung, cervical, breast and prostate carcinomas. In patients with prostate cancer, an increase in the NE cell population correlates with progressive and metastatic tumors, and poor clinical prognosis because of advanced prostate cancer that is androgen insensitive and consequently resistant to traditional modes of chemotherapy.

***The developmental origin of NE cells is uncharacterized.*** The developmental lineage (*i.e*., the nature of the progenitor cells) of prostate carcinoma NE cells, whether of neuronal or epithelial origin, is unknown. NE cells have morphological similarities to neuronal cells and many secrete various neuropeptides including bombesin, serotonin, and thyroid-stimulating hormone-like, parathyroid hormone-like, and calcitonin-like peptides. Moreover, NE cells contain specific neuronal markers including chromogranin A ("Chr A") and neuron-specific enolase ("NSE"). Additionally, NE cells may provide paracrine stimuli for proliferation of the surrounding epithelial cells within the tumor, and thereby facilitate the growth of neighboring cancer cells.

***Involvement of MAP kinase (MAPK) in neuroendocrine differentiation in cancer cells.*** The precise mechanism by which the above-mentioned uncharacterized progenitor cells differentiate into NE cells has not been determined. NE differentiation in prostate cancer cells may be facilitated by an increase in cellular cAMP. For example, addition of cAMP analogs, or increases in cellular cAMP that activate cAMP-dependent Protein Kinase ("PKA") induce NE differentiation in prostate cancer cell lines *in vitro.* Thus, activation of PKA and downstream signaling pathways, such as MAP Kinase ("MAPK") may be involved, but the downstream effectors of this proposed MAPK-mediated process have largely not been identified either *in vivo* or *in vitro.*

MAPK is involved centrally involved in multiple signal transduction pathways leading to activation of many transcription factors involved in cell proliferation, differentiation, apoptosis and adaptation to stress. The precise physiological/biological outcome depends on the nature of the expression and phosphorylation of upstream activators in the particular underlying signal transduction pathway.

For example, in fibroblasts, smooth muscle cells and adipocytes, an increase in cellular cAMP inhibits growth by suppression of the MAPK pathway through members of the Ras protein family, whereas in PC12 pre-neuronal cells (derived from rat adrenal medullary), activation of PKA results in growth arrest and differentiation through the Rap1/B-Raf/Mek/MAPK pathway, and addition of Nerve Growth Factor ("NGF") augments this morphological process. More specifically, neuronal differentiation of PC12 cells involves phosphorylation by PKA of active sites on a small GTP-binding protein (Rap 1), subsequent activation of a serine/threonin kinase (*B-Raf*) and the MAPK kinase (*Mek*)*,* which leads to activation of MAPK. This latter pathway is also implicated in the NE differentiation of specific prostate cancer cell lines, such as LNCaP and PC-3M.

Thus, in these examples, two independent mechanisms of growth and differentiation via the same downstream MAPK mediator involve complicated specific MAPK modules, comprised of unique proteins, that program a cell for quiescence or differentiation. Additionally, it is likely, that a cell must first over-ride alternate mitogenic pathways by induction of particular proteins. However, the precise mechanism by which currently uncharacterized progenitor cells differentiate into NE cells is unknown, as is the underlying signal transduction pathway involved in neuroendocrine differentiation.

Thus, there is a need in the art to characterize the progenitors of NE cells. Moreover, there is a need in the art to understand the mechanisms by which such progenitors differentiate into NE Cells. Furthermore there is a need in the art to identify and characterize the mediators of signal transduction pathways that are involved in these mechanisms.

***The insulin-like growth factor signaling system, and the "IGFBP superfamily.* The** insulin-like growth factor (hereinafter "IGF") signaling system (hereinafter "IGF axis") is comprised of the ligands IGF-I, IGF-II and insulin, and a family of transmembrane receptors including the insulin, type 1 and type 2 IGF receptors (Daughaday and Rotwein, *Endocr. Rev.* 10:68-9, 1989; Werner et al., "Insulin-like growth factors: Molecular and Cellular Aspects," CRC Press, LeRoith (eds.), Boca Raton, pp. 17-47, 1991; Baxter & Martin, *Prog. Growth Factor Res.* 1:49-68, 1989).

Additionally, the IGF axis includes the human IGFBP superfamily, comprising six high-affinity IGF binding species (hereinafter "IGFBPs"), and nine low-affinity IGFBP-related proteins (hereinafter "IGFBP-rPs") (summarized in Table 1). Six IGFBPs have been identified, cloned and sequenced (Baxter & Martin, *Prog. Growth Factor Res.* 1:49-68, 1989; Rosenfeld *et al., Recent Progress Hormone Res.* 46:99-159,1991; Shimasaki & Ling, *Prog. Growth Factor Res.* 3:243-266, 1991). The IGFBPs share a high degree of similarity in their primary protein structure, particularly in the corresponding N- and C-terminal regions, which are separated by a variable mid-protein segment of 55 to 95 amino acid residues (Shimasaki & Ling, *Prog. Growth Factor Res.* 3:243-266, 1991). The IGFBPs bind IGF-I and IGF-II, but not insulin, with high affinity (Jones & Clemmons, *Endocr. Rev.* 16:3-34,1995). The IGFBPs appear to serve essential functions of transporting the IGFs, prolonging IGF half lives, and regulating the availability of free IGFs for interaction with IGF receptors. Accordingly, the IGFBPs modulate the effects of IGFs on growth and differentiation (Jones & Clemmons, *Endocr. Rev.* 16:3-34, 1995; Lowe, "Insulin-like growth factors: Molecular and Cellular Aspects," CRC Press, LeRoith (eds.), Boca Raton, pp. 49-85, 1991; Oh et al., *Growth Regul.* 3: 113-123, 1993; Kelley et al., *Int. J. Biochem. Cell Biol.* 28:619-637, 1993; Rajaram et al., *Endocr. Rev.* 18:801-831, 1997). Recent data is also consistent with a biological mechanism whereby some IGFBPs (*e.g*., IGFBP-3) may be important growth-suppressing factors in various cell systems through an IGF-independent mechanism (Oh, *Endocrine.* 7:111-113, 1997; Oh, *Breast Cancer Res. Treat.* 47:283-293, 1998).

The "IGFBP-rPs," which bind IGF with relatively low affinity, nonetheless have a significant similarity to the IGFBPs in their N-terminal domains (Hwa et al., *Acta Ped. Scand.* 428:37-45, 1999) (Table 1). Thus, current data supports the broad concept of an "IGFBP superfamily" with both high- and low-affinity members, wherein at least some members influence cell growth and differentiation by both IGF-dependent and IGF-independent means (Hwa et al., *Acta Ped. Scand.* 428:37-45, 1999; Baxter et al., *Endocrinology* 139:4036, 1998; Baxter et al., *J*. *Clin. Endocrinol. Metab.* 83:3213, 1998).

***lnsulin-like Growth Factor Binding Protein-Belated Protein 1 ("IGFBP-rP1").*** IGFBP-rP1was first isolated and cloned from leptomeningal and senescent human mammary epithelial cells, respectively. The amino acid sequence of the IGFBP-rP1 protein was shown to have similarity to the high affinity IGF binding proteins, IGFBP 1-6. Recombinant IGFBP-rP1 peptide specifically binds IGF I and II, but with low affinity relative to IGFBP 1-6. While the principal role of at least some of the high-affinity IGFBPs, as described above, has been postulated to be in transporting IGFs and protecting them from rapid clearance and/or degradation, the biological function of IGFBP-rP1 is unknown.

*The IGF axis is important in cancer.* The IGFs are major regulators of mammary epithelial and breast cancer cell growth (Jones & Clemmons, *Endocr. Rev.* 16:3-34, 1995; Oh, *Endocrine.* 7:111-113, 1997; Oh, *Breast Cancer Res. Treat.* 47:283-293, 1998; De Leon et al., *Growth Factors* 6:327-336,1992; Furlanetto & DiCarlo, *Cancer Res.* 44:2122-2128, 1984; Huff et al., *Cancer Res.* 46:4613-4619, 1986). For example, IGF-I and IGF-II are potent mitogens for a number of breast cancer cell lines *in vitro* (De Leon et al., *Growth Factors* 6:327-336, 1992; Huff et al., *Cancer Res.* 46:4613-4619, 1986; Westley & May, *Reviews on Endocrine-Related Cancer* 39:29-34, 1991; Baxter et al., Insulin-like Growth Factors/Somatomedins, De Gruyter, Spencer (ed.), Berlin, pp615-618, 1983). Moreover, IGF-I and IGF-II mRNAs are detectable in the majority of human breast tumor specimens (Cullen et al., *Cancer Res.* 51:4978-4985, 1991; Paik, *Breast Cancer Res. Treat.* 22:31-38, 1992). Virtually all breast tumor specimens, and cell lines derived therefrom, express and produce type-1 and type-2 IGF receptors, and insulin receptors (Cullen et. al., *Cancer Res.* 50:48-53, 1990; Bonneterre et al., *Cancer Res.* 50:6931-6935, 1990; Peyrat & Bonneterre, *Breast Cancer Res. Treat.* 22:59-67, 1992; De Leon et. al., *Biochem. Biophys. Res. Commun.* 152:398-405, 1988). The mitogenic effects of both IGF-I and IGF-II are mediated by the type-1 IGF receptor, as determined through the use of estrogen-dependent breast cancer cells (De Leon et al., *Growth Factors* 6:327-336, 1992; Papa et. al., *Mol. Endocrinol.* 5:709-717, 1991).

In contrast, relatively little is known about the molecular mechanisms and biological functions of the IGFBPs in the context of breast cancer. Specifically, breast cancer cells are known to secrete various types of IGFBPs, and these appear to regulate the availability of free IGFs for interaction with IGF receptors (Lamson et al., *Growth Factors* 5:19-28, 1991). The predominant secreted IGFBP appears to correlate with the estrogen receptor status of the cell (Figueroa et al., *J. Cell. Biochem.* 52:196-205, 1993). Estrogen-nonresponsive (estrogen receptor (ER)-negative) cells secrete predominantly IGFBP-3 and IGFBP-4 as major species, and IGFBP-6 as a minor one. Estrogen-responsive (ER-positive) cells secrete IGFBP-2 and IGFBP-4 as major species, and IGFBP-3 and IGFBP-5 as minor ones.

***The need for additional discovery.*** Therefore, there is a need in the art to understand the biological functions of IGFBP-rPs, and the related enabling mechanisms. Additionally, there is a need in the art to determine how these mechanisms and proteins can be modulated and used for prognostic, diagnostic and therapeutic applications in cancer treatment. Moreover, there is a need in the art to characterize the progenitors ofNE cells. Furthermore, there is a need in the art to understand the mechanisms by which such progenitors differentiate into NE Cells. Finally, there is a need in the art to identify and characterize the mediators of signal transduction pathways that are involved in these mechanisms.

**Table 1. Structural Characteristics of the Human IGFBP Superfamily**

| **IGFBP** | **Mol. Weight (kD)** | **Number of amino acids** | **Number of cysteines** | **N-linked glycosylation** | **Chromosomal localization** | **mRNA size (kb)** |
|---|---|---|---|---|---|---|
| *High affinity IGFBP proteins* | | | | | | |
| IGFBP-1 | 25.271 | 234 | 18 | No | 7p | 1.6 |
| IGFBP-2 | 31.355 | 289 | 18 | No | 2q | 1.5 |
| IGFBP-3 | 28.717 | 264 | 18 | Yes | 7p | 2.4 |
| IGFBP-4 | 25.957 | 237 | 20 | Yes | 17q | 1.7 |
| IGFBP-5 | 28.553 | 252 | 18 | No | 2q | 1.7,6.0 |
| IGFBP-6 | 22.847 | 216 | 16 | No | 12 | 1.1 |

| *Low affinity IGFBP related proteins* | | | | | | |
|---|---|---|---|---|---|---|
| IGFBP-rP1 | | 251 | 18 | Yes | 4q | 1.1 |
| IGFBP-rP2 | | 349(pre) | 39 | Yes | 6q | 2.4 |
| IGFBP-rP3 | | 357 (pre) | 41 | ?(No) | 8q | 2.4 |
| IGFBP-rP4 | | 379 (pre) | 35 | ?(No) | ? | 2.4 |

### SUMMARY OF THE INVENTION

The present invention provides an isolated DNA sequence encoding the protein of SEQ ID NO:1.

The present invention further provides an isolated protein or polypeptide of SEQ ID NO:1.

The present invention further provides an in vitro cancer diagnostic assay for determining a progressive cancer, comprising: (a) obtaining a sample of a tumor tissue; and (b) performing a sequence identity assay to determine an amount of protein sequences expressed by the cDNA sequence described in SEQ ID NO: 1 or expressed by a sequence having at least 90% homology with the coding region of SEQ ID NO:1 present in the sample, whereby the amount of the said protein sequences is correlated with progressive cancer. Preferably, the sequence identity assay is selected from the group consisting of PCR assays, ELTSA immunologic assays, hybridization assays, and combinations thereof.

The present invention further provides a method for identifying test compounds having cancer therapeutic activity, comprising: (a) contacting a test compound with a cell expressing both a IGFBP-rP1 polypeptide, and a IGFBP-rP1 interacting protein being expressed by the cDNA sequence described in SEQ ID NO:1 or expressed by a sequence having at least 90% homology with the coding region of SEQ ID NO:1, wherein either the IGFBP-rP1, or the IGFBP-rP1 interacting protein, are recombinantly expressed; and (b) determining whether the test compound alters growth rate or differentiation of the cell, as determined by the presence or absence of specific markers of cellular differentiation, whereby test compounds that alter the growth rate or differentiation of the cell are identified as cancer therapeutic compounds.
Preferably, the IGFBP-rP1 interacting protein is P25.1.

The present invention further provides a method for identifying test compounds having cancer therapeutic activity, comprising: (a) contacting a functional IGFBP-rP1 in the presence and absence of a test compound with a functional IGFBP-rP1 interacting protein according to SEQ ID NO:1, and determining whether the test compound inhibits the interaction between the IGFBP-rP1 interacting protein and the IGFBP-rP1; (b) further contacting the test compounds of (a), that inhibit said interaction, with a cell expressing both a IGFBP-rP1 polypeptide, and a IGFBP-rP1 interacting protein, wherein either the IGFBP-rP1, or the IGFBP-rP1 interacting protein, are recombinantly expressed; and (c) determining whether the test compound alters growth rate or differentiation of the cell, as determined by the presence or absence of specific markers of cellular differentiation, whereby test compounds that alter the growth rate or differentiation of the cell are identified as cancer therapeutic compounds. Preferably, the IGFBP-rP1 interacting protein is P25.1.

The present invention further provides an in vitro method for identifying test compounds having cancer therapeutic activity, comprising: (a) contacting a test compound with a cell expressing both a IGFBP-rP1 polypeptide, and a IGFBP-rP1 interacting protein being expressed by the cDNA sequence described in SEQ ID NO:1 or expressed by a sequence having at least 90% homology with the coding region of SEQ ID NO:1; and (b) measuring the extent of nuclear translocation of either the IGFBP-rP1 interacting protein, or the IGFBP-rP1 polypeptide, whereby test compounds that alter the extent of nuclear translocation of either the IGFBP-rP1 interacting protein, or the IGFBP-rP1 are identified as cancer therapeutic compounds. Preferably, the IGFBP-rP1 interacting protein is P25.1.

The present invention further provides an in vitro method for identifying test compounds having cancer therapeutic activity, comprising: (a) contacting a test compound with a cell expressing both a IGFBP-rP1 polypeptide, and a IGFBP-rP1 interacting protein being expressed by the cDNA sequence described in SEQ ID NO:1 or expressed by a sequence having at least 90% homology with the coding region of SEQ ID NO:1; and (b) measuring phosphorylation or activation status of an intracellular protein, wherein the intracellular protein is Ras, PKA, RAP1, B-Raf; Mek, or MAPK, whereby test compounds that alter the phosphorylation or the activation status of a said intracellular protein are identified as cancer therapeutic compounds. Preferably, the IGFBP-rP1 interacting protein is P25.1.

The present invention further provides an in vitro method for identifying test compounds having cancer therapeutic activity, comprising: (a) contacting a test compound with a functional IGFBP-rP1 interacting protein according to SEQ ID NO:1, and determining whether the test compound interacts with the functional IGFBP-rP1 interacting protein; (b) further contacting the test compounds of (a), that interact with the functional IGFBP-rP1 interacting protein, with a cell expressing both a IGFBP-rP1 polypeptide, and a IGFBP-rP1 interacting protein being expressed by the cDNA sequence described in SEQ ID NO:1 or expressed by a sequence having at least 90% homology with the coding region of SEQ ID NO:1, wherein either the IGFBP-rP1, or the IGFBP-rP1 interacting protein, are recombinantly expressed; and (c) determining whether the test compound alters growth rate or differentiation of the cell, as determined by the presence or absence of specific markers of cellular differentiation, whereby test compounds that alter the growth rate or differentiation of the cell are identified as cancer therapeutic compounds. Preferably, the IGFBP-rP1 interacting protein is P25.1.

The present invention further provides an in vitro method for identifying test compounds having cancer therapeutic activity, comprising: (a) contacting a test compound with a cell or cell lysate containing mRNA sequences of IGFBP-rP1 interacting protein, the said protein being expressed by the cDNA sequence described in SEQ ID NO:1 or expressed by a sequence having at least 90% homology with the coding region of SEQ ID NO:1; and (b) detecting translational inhibition of the IGFBP-rP1 interacting protein transcripts. Preferably, the IGFBP-rP1 interacting protein is P25.1.

The present invention further provides an antagonist of biological activity of the protein according to SEQ ID NO:1, wherein the antagonist is selected from the group consisting of an inhibitor of the interaction between IGFBP-rP1 and the said protein, an antibody against the said protein, an antisense molecule against the cDNA sequence of SEQ ID NO:1 or against a sequence having at least 90% homology with the coding region of SEQ ID NO:1, and a ribozyme molecule. Preferably, the antisense molecule is a specific antisense oligonucleotide of at least 10 bases complementary to the cDNA sequence SEQ ID NO:1. Preferably, the antisense oligonucleotide is a 15-25 base oligonucleotide incorporating an oligonucleotide sequence selected from the group consisting of SEQ ID NOS:2-16; and combinations thereof. Preferably, the IGFBP-rP1 interacting protein is P25.1.

The present invention further provides an anticancer pharmaceutical composition comprising a therapeutically effective amount of an antagonist as described above, and a pharmaceutically acceptable carrier.

The present invention further describes a method for treating or preventing cancer in a subject needing treatment comprising administering to the subject in which such treatment or prevention is desired a therapeutically effective amount of an antagonist as described above. Preferably, the cancer to be treated or prevented is small cell lung, cervical, breast, or prostate carcinoma.

The present invention further provides an anticancer pharmaceutical composition comprising an antibody that binds to a protein according to SEQ ID NO:1 or a binding fragment thereof, and a pharmaceutically acceptable carrier. Preferably, the antibody is a humanized monoclonal antibody.

### BRIEF DESCRIPTION OF THE FIGURES

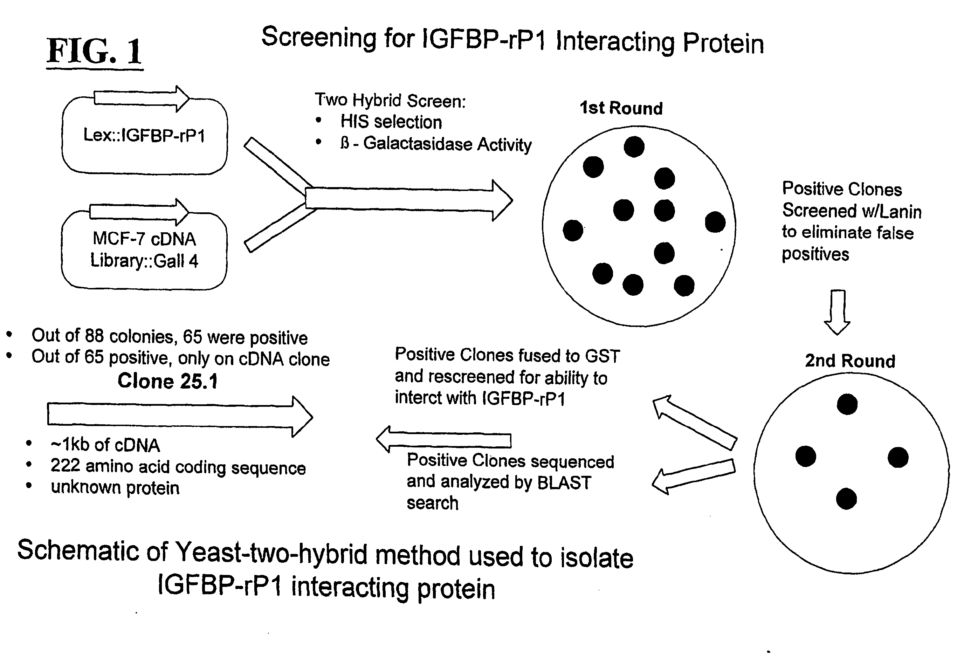

Figure 1 shows a schematic diagram of the isolation process for isolating IGFBP-rP1 interacting protein using the yeast-two-hybrid method. A novel cDNA clone, 25.1, was isolated using full-length human IGFBP-rP1 and a cDNA library from MCF-7 breast cancer cells. The cDNA encodes a novel protein of 223 amino acids with a putative nuclear localization signal and a possible myristylation site.

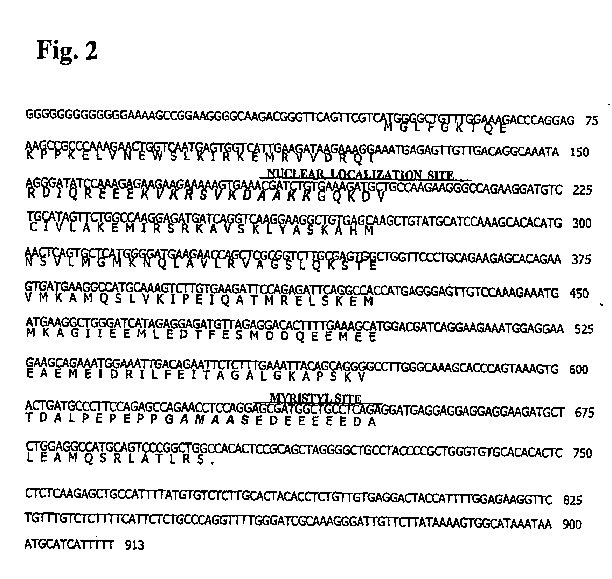

Figure 2 shows the sequence of the novel P25.1 cDNA. The cDNA encodes a novel protein of 222 amino acids with a putative nuclear localization signal and a possible myristylation site (both marked in the figure above the corresponding nucleotide sequence).

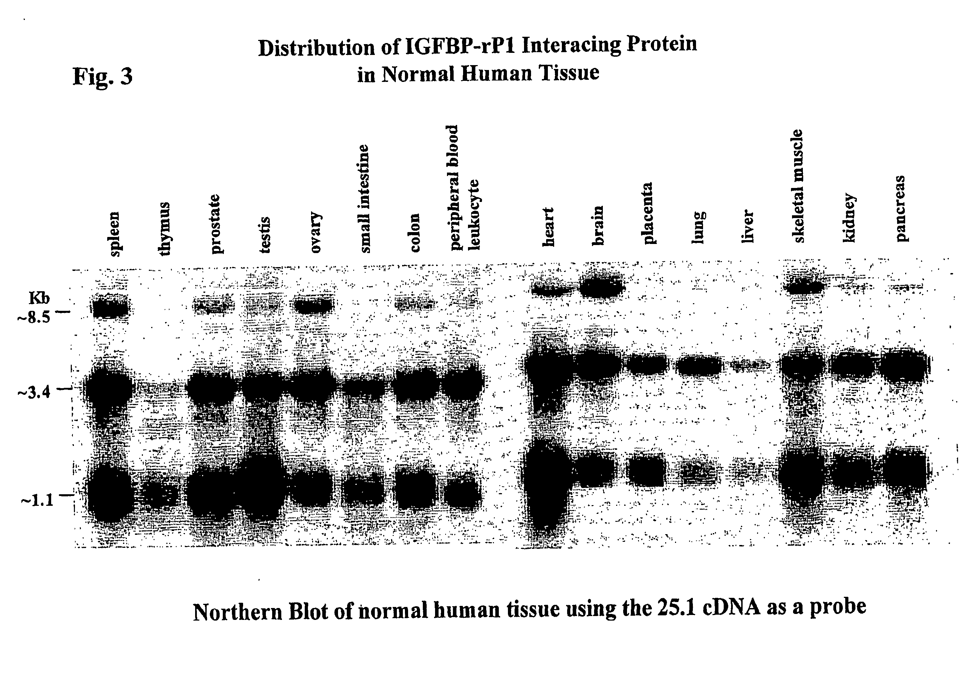

Figure 3 shows a Northern blot of normal human tissue showing differential expression of three species of P25.1 mRNA. A Northern blot of normal human tissue with 2µg of mRNA per lane was hybridized with a [³²P]-α-dCTP labeled 25.1 cDNA probe. Tissues represented in the lanes are as follows: Lane 1, spleen; Lane 2, thymus; Lane 3, prostate; Lane 4, testis; Lane 5, ovary; Lane 6, small intestine; Lane 7, colon; Lane 8, peripheral blood leukocyte; Lane 9, heart; Lane 10, Brain; Lane 11, placenta; Lane 12, lung; Lane 13, liver; Lane 14, skeletal muscle; Lane 15, kidney; Lane 16, pancreas.

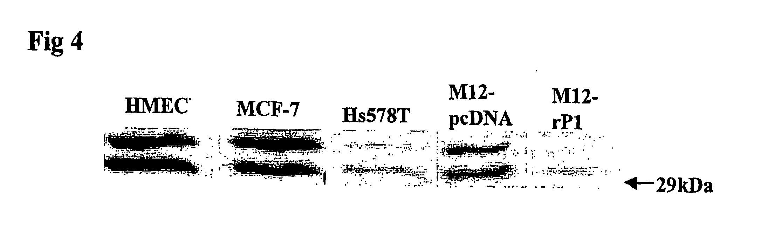

Figure 4 shows the detection of endogenous P25.1 protein by Western immunoblotting of cell lysates from breast and prostate cancer cell lines. P25.1 protein was found in reduced amount in cancer cells relative to normal cells. Cell lysates were prepared in RIPA Buffer (150 mM NaCl, 20 mM Hepes pH 7.5, 1 % Triton-100, 1% sodium deoxycholate, 0.1% SDS, with protease inhibitor cocktail) from confluent dishes of HMEC (primary mammary epithelial cells), MCF-7 and Hs578T breast cancer cells, and M12-pcDNA and M12-rP1 stable prostate cancer cell lines. Equal amounts of protein were resolved on SDS:PAGE, and the P25.1 protein detected by Western immunoblotting with affinity-purified anti-P25.1 polyclonal antibody.

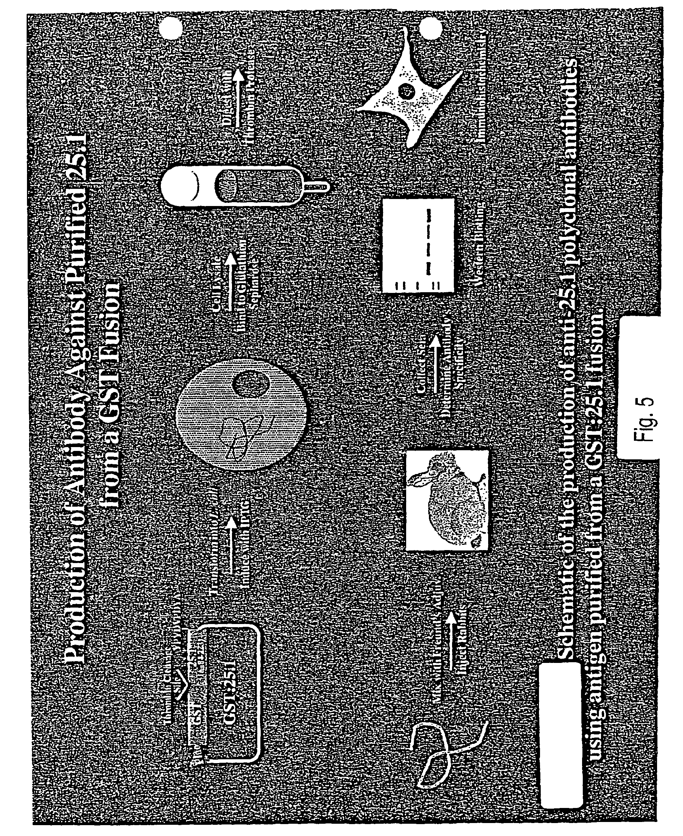

Figure 5 shows a schematic diagram of expression and purification of P25.1 protein, and production of polyclonal antibody. Briefly, a fusion protein between the P25.1 cDNA and GST was generated. The GST:P25:1 fusion was transformed into *E. coli* that were grown to log phase, induced with IPTG, and then lysed in PBS by sonication. The fusion protein was bound to glutathione Sepharose and the P25.1 protein released by digestion with thrombin. The purified protein was mixed with Freund's Adjuvant and injected subcutaneously into New Zealand White female rabbits, with two subsequent boosts. The Rabbits were exsanguinated and sera were collected.

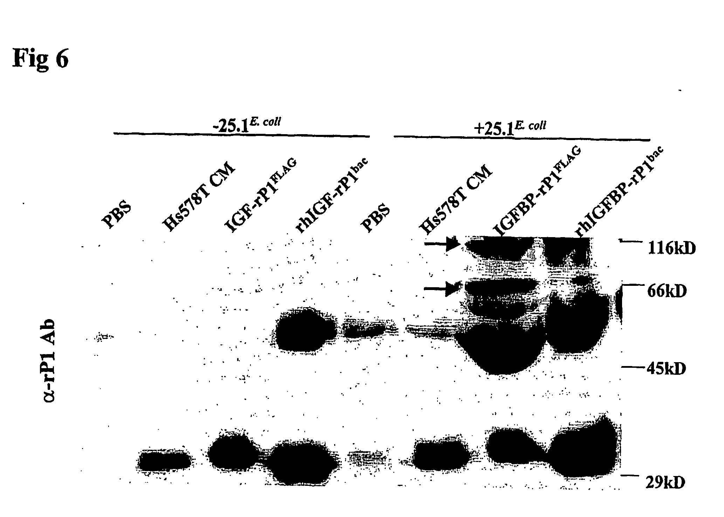

Figure 6 shows confirmation of the interaction between IGFBP-rP1 and P25.1. Purified P25.1 *E*. *coli* and various sources of IGFBP-rP1 were used for affinity crosslinking to demonstrate a physical association between the two proteins. Purified P25.1 (expressed in *E. coli*) and various sources of IGFBP-rP1 were employed for affinity crosslinking. The crosslinked products were resolved through the use of 12% SDS:PAGE, transferred to nitrocellulose and immunoblotted with anti-IGFBP-rP1. IGFBP-rP 1 were obtained from: conditioned media (CM) from Hs578T breast cancer cells (endogenous source); CM from transiently expressed IGFBP-rP1FLAG in COS-7 cells; and affinity purified IGFBP-rP1FLAG from recombinant infected insect cells and then chemically crosslinked with DSS in the presence (right panel) or absence (left panel) of *E. coli-*derived, purified P25.1.

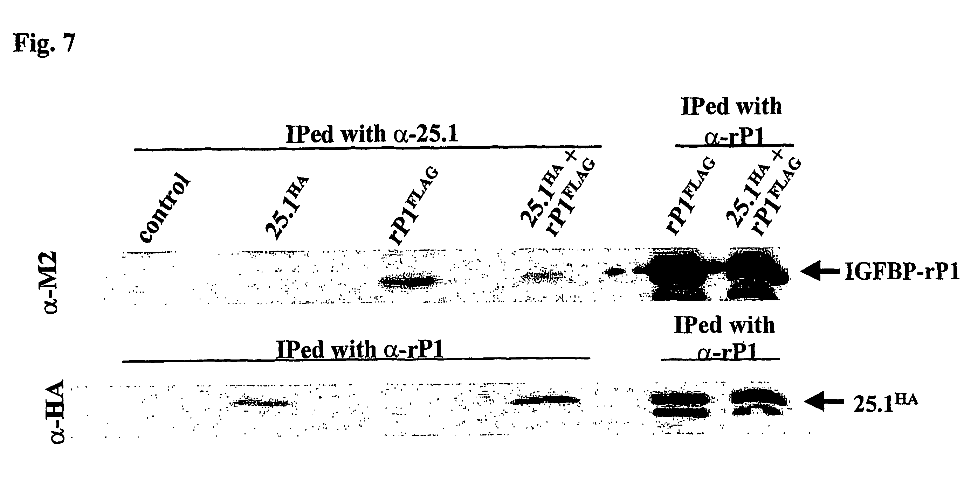

Figure 7 shows co-immunoprecipiations (''Iped'') of P25.1^{HA} and IGFBP-rP1^{FLAG} from transfected COS-7 cells. COS-7 cells were transfected with pcDNA3, pcDNA3:25.1HA, pcDNA3.1:IGFBP-rP1FLAG, or both pcDNA3:25.1HA and pcDNA3.1:IGFBP-rPIFLAG. After 48 hours, cells were harvested and lysed by sonication in PBS with protease inhibitors. Extracts were immuno-precipitated with anti-IGFBP-rP1 (right two lanes) or anti-P25.1 (left four lanes), after each had been pre-absorbed to Protein A-Sepharose 4 Fast Flow.

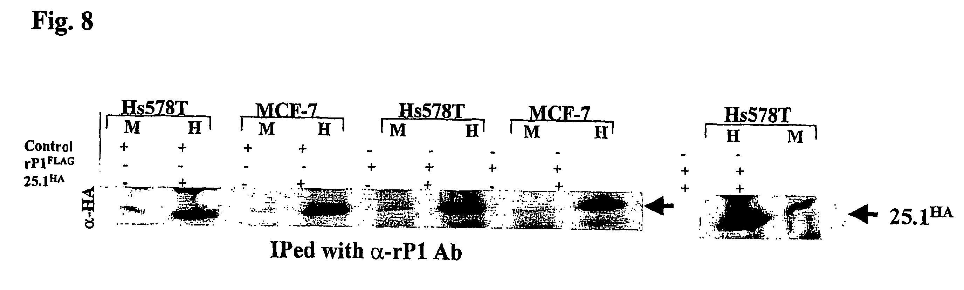

Figure 8 shows *in vitro* mixing and co-immunprecipitation of P25.1^{HA} and IGFBP-rP1^{FLAG} from transfected MCF-7 (M) or Hs578T (H) cells. MCF-7 (M) or Hs578T (H) cells were transfected with pcDNA3 (control), pcDNA3:25.1HA (25.1^{HA}), pcDNA3.1:IGFBP-rP1^{FLAG} (rP1FLAG), or pcDNA3:25.1^{HA} + pcDNA3.1:IGFBP-P1^{FLAG}. Equal volumes of CM from the independent transfections were mixed and immunoprecipitated ("Iped") with anti-IGFBP-rP1, preabsorbed to Protein A-Sepharose 4 Fast Flow. The immunoprecipitation products were separated through the use of 12% SDS:PAGE, transferred to nitrocellulose and immuno-blotted with anti-HA rat monoclonal antibody.

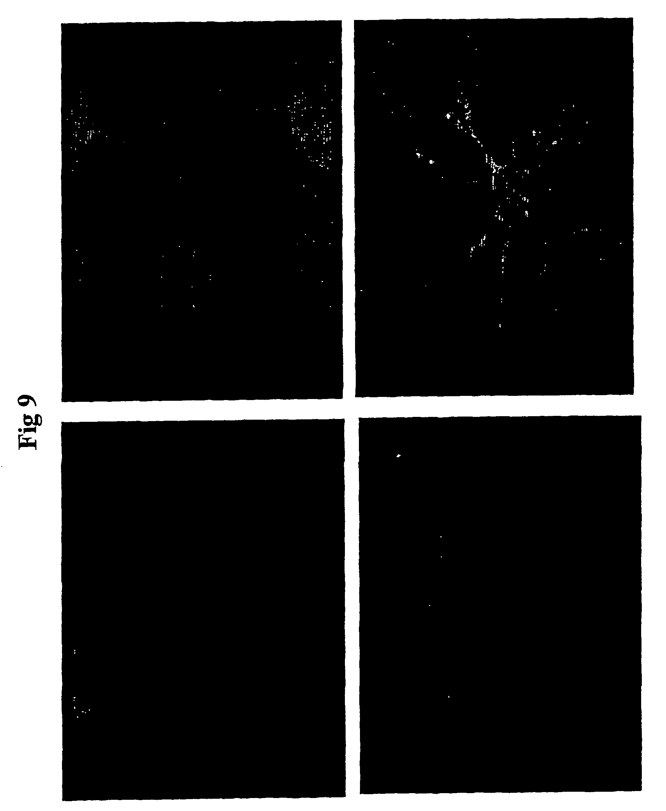

Figure 9 shows neuroendocrine-like differentiation of M12:rP1 stable transfected cells transiently expressing P25.1HA. The M12:rP1 cells over-expressing P25.1protein showed a marked increase in neuroendocrine differentiation. pcDNA3:25.1HA was transiently transfected into the M12:pcDNA3 or M12:IGFBP-rP1 stable transfected lines using Fugene 6 transfection reagent. Transient transfected cells were trypsinized and re-plated into 12-well dishes. Endogenous and transiently-expressed 25.1 proteins were examined using immuncytochemistry with either anti-P25.1 Ab or anti-HA mAb (monoclonal antibody to the HA epitope). Those M12:rP1 cells over-expressing 25.1 protein showed a marked increase in neuroendocrine differentiation. Thus, transiently expressing P25.1^{HA} in prostate cancer cell lines, stably, over-expressing IGFBP-rP1, facilitates neuroendocrine differentiation.

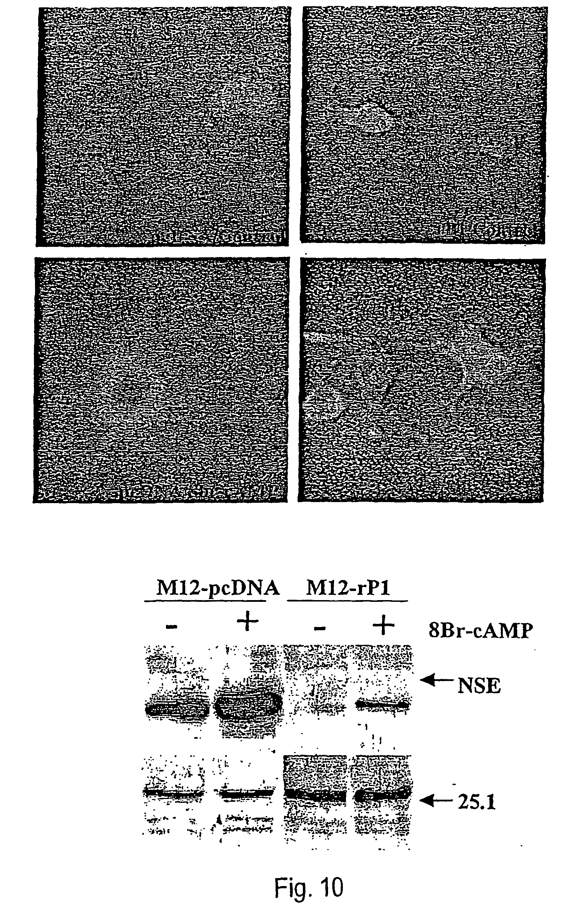

Figure 10 shows P25.1 protein over-expressing M12:rP1 stables stain positive with neuroendocrine marker and expression of transient P25.1 is increased in the presence of 8br-cAMP. Transient tranfections of M12:pcDNA or M12:rP1 with pcDNA3 or pcDNA3:25.1HA were seeded into 6-well or 24-well dishes and after attachment were incubated -/+ 0.5 µM 8br-cAMP. Total cell lysates from the 6 well dishes were prepared in 0.25 ml of RIPA buffer, equal quantities of protein were resolved on a 12% SDS:PAGE, transferred to nitrocellulose and blotted with affinity purified anti-P25.1 polyclonal antibody and anti-NSE monoclonal antibody. The cells were co-stained with anti-P25.1 Ab and anti-Chromogranin A mAb, as the primary antibodies. FITC conjugated, anti-rabbit IgG (green) and Alexa conjugated anti-mouse (red) were used to detect P25.1 or Chromogranin A, respectively by immunofluorescence. The independent images of the same field were overlayed and co-expression is depicted in yellow. The M12:rP1 stables cells which transiently over-express P25.1, stained positive with the neuroendocrine marker Chromogranin A.

### DETAILED DESCRIPTION OF THE INVENTION

### Definitions:

"**P25.1**" means P25.1 gene products, such as transcripts and the P25.1 protein. Polypeptides or peptide fragments of the P25.1 are referred to as P25.1 polypeptides or P25.1 peptides. Fusions of P25.1, or P25.1 polypeptides, or peptide fragments to an unrelated protein are referred to herein as P25.1 fusion proteins. A functional P25.1 refers to a protein that binds IGFBP-rP1, or IGFBP-rP1 peptides *in vivo* or *in vitro.*

**"P25.1 nucleotides or coding sequences":** means DNA sequences encoding P25.1 mRNA transcripts, P25.1 protein, polypeptide or peptide fragments of P25.1, or P25.1 fusion proteins. P25.1-nucleotide sequences encompass DNA, including genomic DNA (e.g., the P25.1 gene) or cDNA.

**"IGFBP-rP1"** means IGFBP-rP1 gene products, *e.g.,* transcripts and the IGFBP-rP1 protein. Polypeptides or peptide fragments of the IGFBP-rP1 are referred to as IGFBP-rP1 polypeptides or IGFBP-rP1 peptides. Fusions of IGFBP-rP1, or IGFBP-rP1 polypeptides, or peptide fragments to an unrelated protein are referred to herein as IGFBP-rP1 fusion proteins. A functional IGFBP-rP1 refers to a protein that binds P25.1, or P25.1 peptides *in vivo* or in *vitro.*

**"NE"** means "neuroendocrine."

**"HA"** means "the epitope tag YPYDVPDYA."

**"NLS"** means "nuclear localization signal."

### Overview

A Yeast-Two-Hybrid system was employed using a cDNA library from MCF-7 breast cancer cells to further elucidate the biological role of IGFBP-rP1. The present invention provides a novel cDNA (25.1) that encodes a novel protein, P25.1, that interacts specifically with IGFBP-rP1, and leads to NE differentiation of a prostate cancer cell line over-expressing the IGFBP-rP1 protein.

The present invention encompasses screening methods (e.g., assays) for identification of cancer therapeutic compounds. The invention further encompasses agonists and antagonists of P25.1, including small molecules, large molecules, and antibodies, as well as nucleotide sequences that can be used to inhibit P25.1 gene expression, such as antisense and ribozyme molecules. The present invention further encompasses the use of such compounds to treat cancer and to induce neuroendocrine differentiation in cancer and other cells.

In particular, cellular and non-cellular assays are described that can be used to identify compounds that interact with P25.1, and/or IGFBP-rP1, to antagonize association between these two molecules, and/or to modulate the activity of P25.1, IGFBP-rP1, or the P25.1:IGFBP-rP1complex. The cell based assays can be used to identify compounds or compositions that affect the activity, nuclear translocation, or cellular compartmentalization of P25.1, IGFBP-rP1, or the P25.1:IGFBP-rP1complex, whether they bind to of P25.1, IGFBP-rP1, or the P25.1:IGFBP-rP1complex or act on intracellular factors involved in the downstream signal transduction pathway. Such cell-based assays of the invention utilize cells, cell lines, or engineered cells or cell lines that express P25.1, and/or IGFBP-rP1. The cells can be further engineered to incorporate a reporter molecule linked to the signal transduced by P25.1, IGFBP-rP1, or the P25.1:IGFBP-rP1 complex to aid in the identification of compounds that modulate P25.1-mediated signaling activity.

The present invention also encompasses the use of cell-based assays or cell-lysate assays (*e*.*g*., in vitro transcription or translation assays) to screen for compounds or compositions that modulate P25.1 gene expression. For example, such assays could be used test the activity of triple-helix polynucleotides. Alternatively, engineered cells or translation extracts can be used to screen for compounds (including antisense and ribozyme constructs) that modulate the translation of P25.1 mRNA transcripts, and therefore, affect expression of P25.1.

The present invention also provides P25.1 proteins, polypeptides (including soluble P25.1 polypeptides or peptides) and P25.1 fusion proteins for use in cell, and non-cell based assays for screening compounds that interact with, and/or modulate the activity of P25.1, IGFBP-rP1, or the P25.1:IGFBP-rP 1 complex, for use in generating antibodies, and for diagnostics and therapeutics. P25.1 protein products can be used as P25.1 antagonists or agonists to treat cancer or regulate neuroendocrine differentiation. Such P25.1 protein products include but are not limited to peptides or polypeptides corresponding to one or more P25.1 domains (*e.g*., the IGFBP-rP1 interaction domain), truncated P25.1 polypeptides lacking one or more P25.1 domains (*e.g*., lacking the nuclear translocation signal, or the myristilation site), and P25.1 fusion protein products (*e.g.*, GST fusions, or eptitope-tagged fusions). Alternatively, antibodies to the P25.1 that are P25.1 antagonists or agonists (including compounds that modulate signal transduction that may act on downstream targets in the P25.1 signal transduction pathway) can be used to treat cancer and regulate neuroendocrine differentiation.

For example, the administration of an effective amount of an appropriate soluble P25.1 polypeptide, or a P25.1 fusion protein (*e.g*., P25.1^{HA}) would interact with and thereby alternatively "mop up," "neutralize," or "activate" endogenous IGFBP-rP1, to modulate the activity of P25.1, IGFBP-rP1 or the P25.1/IGFBP-rP1complex. In yet another approach, nucleotide constructs encoding such P25.1 products can be used to genetically engineer host cells to express such P25.1 products *in vivo;* these genetically engineered cells can function as "bioreactors" in the body, delivering a continuous supply of the appropriate P25.1, P25.1 peptide, soluble P25.1 polypeptide, or P25.1 fusion protein that will alternatively "mop up," neutralize, or activate IGFBP-rP1.

"Gene therapy" approaches for the modulation of P25.1 expression and/or activity in the treatment of cancer, or for modulating neuroendocrine differentiation are within the scope of the invention. For example, nucleotide constructs encoding functional P25.1, mutant P25.1, as well as antisense and ribozyme molecules can be used to regulate P25.1 expression. The invention also encompasses pharmaceutical formulations and methods for treating cancer and modulating neuroendocrine differentiation.

### Cloning of a Novel IGFBP-rP1 Interacting Protein, P25.1

To elucidate the biological function of IGFBP-rP1, the Yeast-Two Hybrid interactive cloning system was employed using a cDNA library from MCF-7 breast cancer cells to identify novel IGFBP-rP1 interacting proteins (Figure 1). One interacting cDNA clone, P25.1, encoding a partial open reading frame to an unknown gene product was identified. The full-length cDNA product was isolated using the 5' RACE technique. The novel P25.1 cDNA is 913 bp in length encoding a 222 amino acid protein (P25.1) that contains a nuclear localization signal and a possible myristilation site (Figure 2, and SEQ ID NO:1). *See also* "Example 1" below.

### Expression Pattern of P25.1 mRNA in Multiple Human Tissues

A multi-tissue Northern blot was used to elucidate the expression pattern of the P25.1 gene in normal human tissues. These data indicate that P25.1 was expressed to varying degrees in all tissues tested (spleen, thymus, prostate, testis, small intestine, colon, peripheral blood leukocytes, heart, brain, placenta, lung, liver, skeletal muscle, kidney, and pancreas) (Figure 3). Three differentially regulated transcripts, having sizes of 1.3, 3.4, and 8.5 kb, were present. Differences in the expression of particular mRNA species were seen within and among the various human tissues. The mRNA levels of the three transcripts were down-regulated in cancer cells relative to normal cells, indicating that P25.1 mRNA, and P25.1 protein expression are a useful diagnostic marker for assessing malignant progression. *See also* "Example s2 and 3," below.

### P25.1 Proteins, Polypeptides, and Antibodies

P25.1 protein, polypeptides and peptide fragments, mutated, truncated or deleted forms of the P25.1 and/or P25.1 fusion proteins were prepared for a variety of uses, including but not limited to the generation of antibodies, as reagents in diagnostic assays, the identification of other cellular gene products involved in the regulation cancer and NE differentiation, as reagents in assays for screening for compounds that can be used in the treatment cancer, and as pharmaceutical reagents useful in the treatment of cancer.

***Production of P25.1 Polypeptides.*** The deduced amino acid sequences of P25.1 is shown, along with the corresponding cDNA sequence in Figure 2 and SEQ ID NO:1, where predicted nuclear translocation and myristylation domains are denoted by overbars and bold print. Peptides corresponding to one or more domains of the P25.1 (*e.g.,* nuclear translocation domain), truncated or deleted P25.1 *(e.g.,* P25.1 in which one or more regions or domains is deleted) as well as fusion proteins in which the full length P25.1, an P25.1 peptide or truncated P25.1 is fused to an unrelated protein are also within the scope of the invention. Such soluble peptides, proteins, fusion proteins, or antibodies (including anti-idiotypic antibodies) that bind to and 'neutralize' circulating natural ligand for the P25.1, can be used as described herein to treat cancer or modulate NE differentiation. To this end, peptides corresponding to individual domains of P25.1, soluble deletion mutants of P25.1, or the entire P25.1 can be fused to another polypeptide (e.g., an IgFc polypeptide, or eptitope tag).

Such peptides, polypeptides, and fusion proteins can be prepared by recombinant DNA techniques. For example, nucleotide sequences encoding one or more P25.1 regions or domains can be synthesized or cloned and ligated together to encode a soluble P25.1 polypeptide. The DNA sequence encoding one or more P25.1 regions or domains can be ligated together directly or via a linker oligonucleotide that encodes a peptide spacer. Such linkers may encode flexible, glycine-rich amino acid sequences thereby allowing the domains that are strung together to assume a conformation that can bind P25.1 ligands. Alternatively, nucleotide sequences encoding individual regions or domains can be used to express P25.1 peptides.

A variety of host-expression vector systems may be utilized to express nucleotide sequences encoding the appropriate regions of the P25.1 to produce such polypeptides. Where the resulting peptide or polypeptide is a soluble derivative the peptide or polypeptide can be recovered from the culture media. Where the polypeptide or protein is not secreted, the P25.1 product can be recovered from the host cell itself.

The host-expression vector systems also encompass engineered host cells that express the P25.1 or functional equivalents. Purification or enrichment of the P25.1 from such expression systems can be accomplished using appropriate methods well known to those of ordinary skill in the art. However, such engineered host cells themselves may be used in situations where it is important not only to retain the structural and functional characteristics of the P25.1, but to assess biological activity, *e.g*., in drug screening assays.

The host-expression vector systems that may be used for purposes of the invention include but are not limited to microorganisms such as bacteria *(e.g., E. coli, B. subtilis)* transformed with recombinant bacteriophage DNA, plasmid DNA or cosmid DNA expression vectors containing P25.1 nucleotide sequences; yeast *(e.g., Saccharomyces, Pichia)* transformed with recombinant yeast expression vectors containing the P25.1 nucleotide sequences; insect cell systems infected with recombinant virus expression vectors (e.g., baculovirus) containing the P25.1 sequences; plant cell systems infected with recombinant virus expression vectors *(e.g.,* cauliflower mosaic virus, CaMV; tobacco mosaic virus, TMV) or transformed with recombinant plasmid expression vectors (*e.g.*, Ti plasmid) containing P25.1 nucleotide sequences; or mammalian cell systems *(e.g.,* COS, CHO, BHK, 293, 3T3) harboring recombinant expression constructs containing promoters derived from the genome of mammalian cells *(e.g.,* metallothionein promoter) or from mammalian viruses (*e.g*., the adenovirus late promoter; the vaccinia virus 7.5K promoter).

In bacterial systems, a number of expression vectors may be advantageously selected depending upon the use intended for the P25.1 gene product being expressed. For example, when a large quantity of such a protein is to be produced, for the generation of pharmaceutical compositions of P25.1 protein or for raising antibodies to the P25.1 protein, for example, vectors which direct the expression of high levels of fusion protein products that are readily purified may be desirable. Such vectors include, but are not limited, to the *E. coli* expression vector pUR278 (Ruther et al., *EMBO J.* 2:1791, 1983), in which the P25.1 coding sequence may be ligated individually into the vector in frame with the *lacZ* coding region so that a fusion protein is produced; pIN vectors (Inouye & Inouye, *Nucleic Acids Res.* 13:3101-3109, 1985; Van Heeke & Schuster, *J*. *Biol. Chem.* 264: 5503-5509, 1989); and the like. PGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

Alternatively, any fusion protein may be readily purified by utilizing an antibody specific for the fusion protein being expressed. For example, a system described by Janknecht et al. allows for the ready purification of non-denatured fusion proteins expressed in human cell lines (Janknecht, et al., *Proc. Natl. Acad. Sci. USA* 88:8972-8976, 1991). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺:nitriloacetic acid-agarose columns and histidine-tagged proteins are selectively eluted with imidazole-containing buffers.

In an insect system, *Autographa californica nuclear polyhedrosis virus* (AcNPV) is used as a vector to express foreign genes. The virus grows in *Spodoptera frugiperda* cells. The P25.1 coding sequence may be cloned individually into non-essential regions (for example the polyhedrin gene) of the virus and placed under control of an AcNPV promoter (for example the polyhedrin promoter). Successful insertion of P25.1 gene coding sequence will result in inactivation of the polyhedrin gene and production of non-occluded recombinant virus (*i.e.*, virus lacking the proteinaceous coat coded for by the polyhedrin gene). The recombinant viruses are then used to infect cells in which the inserted gene is expressed. (Smith et al., J. *Virol.* 46:584, 1983; Smith, U.S. Pat. No. 4,215,051).

In mammalian host cells, a number of viral-based expression systems may be utilized. In cases where an adenovirus is used as an expression vector, the P25.1 nucleotide sequence of interest may be ligated to an adenovirus transcription/translation control complex, *e.g.,* the late promoter and tripartite leader sequence. This chimeric gene may then be inserted in the adenovirus genome by *in vitro* or *in vivo* recombination. Insertion in a non-essential region of the viral genome *(e.g.,* region El or E3) will result in a recombinant virus that is viable and capable of expressing the P25.1 gene product in infected hosts. (Logan & Shenk, *Proc. Natl. Acad. Sci*. *USA* 81:3655-3659, 1984). Specific initiation signals may also be required for efficient translation of inserted P25.1 nucleotide sequences. These signals include the ATG initiation codon and adjacent sequences. In cases where an entire P25.1 gene or cDNA, including its own initiation codon and adjacent sequences, is inserted into the appropriate expression vector, no additional translational control signals may be needed. However, in cases where only a portion of the P25.1 coding sequence is inserted, exogenous translational control signals, including, perhaps, the ATG initiation codon, must be provided. Furthermore, the initiation codon must be in frame with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (Bittner et al., *Methods Enzyrnol. 153:516-*544, 1987).

In addition, a host cell strain may be chosen that modulates the expression of the inserted sequences, or modifies and processes the gene product in the specific fashion desired. Such modifications (*e.g*., glycosylation) and processing *(e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. Accordingly, eukaryotic host cells that possess cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3 and WI38 cell lines.

For long-term, high-yield production of recombinant proteins, stable expression is preferred. For example, cell lines that stably express the P25.1 sequences may be engineered. Rather than using expression vectors which contain viral origins of replication, host cells can be transformed with DNA controlled by appropriate expression control elements (*e.g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines that express the P25.1 gene product. Such engineered cell lines may be particularly useful in screening and evaluation of compounds that affect the endogenous activity of the P25.1 gene product.

A number of selection systems may be used, including but not limited to the herpes simplex virus thymidine kinase (Wigler et al., *Cell* 11:223, 1977), hypoxanthine-guanine phosphoribosyltransferase (Szybalska & Szybalski, *Proc. Natl. Acad. Sci. USA* 48:2026, 1962), and adenine phosphoribosyltransferase (Low, et al., *Cell* 22:817, 1980) genes can be employed in tk⁻, hgprt⁻ or aprt⁻ cells, respectively. Also, anti-metabolite resistance can be used as the basis of selection for the following genes: *dhfr,* that confers resistance to methotrexate (Wigler et al., *Proc. Natl. Acad. Sci. USA* 77:3567, 1980; O'Hare et al., *Proc. Natl. Acad. Sci. USA* 78:1527,1981); gpt, that confers resistance to mycophenolic acid (Mulligan & Berg, *Proc. Natl. Acad. Sci. USA* 78:2072, 1981); *neo,* that confers resistance to the aminoglycoside G-418 (Colberre-Garapin et al., *J. Mol. Biol.* 150:1, 1981); and *hygro,* that confers resistance to hygromycin (Santerre et al., *Gene* 30:147, 1984).

***Antibodies to P25.1 Polypeptides.*** Antibodies that specifically recognize one or more epitopes of P25.1, or epitopes of conserved variants of P25.1, or peptide fragments of the P25.1 are also encompassed by the invention. Such antibodies include but are not limited to polyclonal antibodies, monoclonal antibodies (mAbs), humanized or chimeric antibodies, single-chain antibodies, Fab fragments, F(ab')₂ fragments, fragments produced by a Fab expression library, anti-idiotypic (anti-Id) antibodies, and epitope-binding fragments of any of the above. The antibodies of the invention may be used, for example, in the detection of the P25.1 in a biological sample and may, therefore, be utilized as part of a diagnostic or prognostic technique whereby patients or tissue samples may be tested for abnormal amounts of P25.1. Such antibodies may also be utilized in conjunction with, for example, compound screening schemes, as described, above, for the evaluation of the effect of test compounds on expression and/or activity of the P25.1 gene product. Additionally, such antibodies can be used in conjunction with the gene therapy techniques described, below, e.g., to evaluate the normal and/or engineered P25.1-expressing cells prior to their introduction into the patient. Such antibodies may additionally be used as a method for the inhibition of abnormal P25.1 activity. Thus, such antibodies may, therefore, be utilized as part of cancer treatment methods.

For the production of antibodies, various host animals may be immunized by injection with the P25.1, an P25.1 peptide (*e.g*., one corresponding the a functional domain of the P25.1, such as nuclear translocation, or IGFBP-rP1 interaction domain), truncated P25.1 polypeptides (P25.1 in which one or more domains, *e.g*., the nuclear translocation or IGFBP-rP 1 interaction domain, has been deleted), functional equivalents of the P25.1 or mutants of the P25.1. Such host animals may include but are not limited to rabbits, mice, hamsters and rats, to name but a few. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and Corynebacterium parvum. Polyclonal antibodies are heterogeneous populations of antibody molecules derived from the sera of the immunized animals. Monoclonal antibodies, which are homogeneous populations of antibodies to a particular antigen, may be obtained by any technique which provides for the production of antibody molecules by continuous cell lines in culture. These include, but are not limited to, the hybridoma technique of Kohler and Milstein, *(Nature* 256:495-497, 1975; and U.S. Pat. 4,376,110), the human B-cell hybridoma technique (Kosbor et al., *Immunology Today* 4:72*,* 1983; Cole et al., *Proc. Natl. Acad. Sci. USA* 80:2026-2030, 1983), and the EBV-hybridoma technique (Cole et al., *Monoclonal Antibodies And Cancer Therapy,* Alan R. Liss, Inc., pp. 77-96, 1985). Such antibodies may be of any immunoglobulin class including IgG, IgM, IgE, IgA, IgD and any subclass thereof. The hybridoma producing the mAb of this invention may be cultivated *in vitro* or *in vivo.* Production of high titers of mAbs *in vivo* makes this the presently preferred method of production.

Additonally, techniques developed for the production of "chimeric antibodies" (Morrison et al., *Proc. Natl. Acad. Sci. USA,* 81:6851-6855, 1984; Neuberger et al., *Nature,* 312:604-608, 1984; Takeda et al., *Nature,* 314:452-454, 1985) by splicing the genes from a mouse antibody molecule of appropriate antigen specificity together with genes from a human antibody molecule of appropriate biological activity can be used. A chimeric antibody is a molecule in which different portions are derived from different animal species, such as those having a variable region derived from a murine mAb and a human immunoglobulin constant region (humanized).

Alternatively, techniques described for the production of single-chain antibodies (U.S. Patent No. 4,946,778; Bird, *Science* 242:423-426, 1988; Huston et al., *Proc. Natl. Acad. Sci. USA* 85:5879-5883, 1988; and Ward et al., *Nature* 334:544-546, 1989) can be adapted to produce single-chain antibodies against P25.1 gene products. Single-chain antibodies are formed by linking the heavy and light chain fragments of the Fv region via an amino acid bridge, resulting in a single-chain polypeptide.

Antibody fragments that recognize specific epitopes may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., *Science* 246:1275-1281, 1989) to allow rapid and easy identification of monoclonal Fab fragments with the desired specificity.

Antibodies to the P25.1 can, in turn, be utilized to generate anti-idiotype antibodies that "mimic" the P25.1, using techniques well known to those skilled in the art (Greenspan & Bona, *FASEB J.* 7:437-444, 1993; and Nissinoff, *J*. *Immunol.* 147:2429-2438,1991). For example antibodies which bind to the P25.1 and competitively inhibit the binding of IGFBP-rP1 to the P25.1 can be used to generate anti-idiotypes that "mimic" the P25.1 and, therefore, bind and neutralize IGFBP-rP1. Such neutralizing anti-idiotypes or Fab fragments of such anti-idiotypes can be used in cancer therapeutic regimens to neutralize the native ligand.

Alternatively, antibodies to P25.1 that can act as agonists of P25.1 activity can be generated. Such antibodies will bind to the P25.1 and activate the signal transducing activity of P25.1 and/or IGFBP-rP1. Such antibodies would be particularly useful for treating particular cancers wherein NE differentiation was a desired event. In addition, antibodies that act as antagonist of P25.1 activity, i.e., inhibit the activation or signaling by P25.1, may be used to treat cancer, and modulate NE differentiation.

### Gene Therapy Approaches to Controlling P25.1 Activity and Treating Cancer

The expression of P25.1 can be controlled *in vivo* (*e.g.,* at the transcriptional or translational level) using gene therapy approaches to regulate P25.1 activity and treat cancer. Certain approaches are described below:

***Gene Replacement Therapy.*** With respect to an increase in the level of normal P25.1 gene expression and/or P25.1 gene product activity, P25.1 nucleic acid sequences can be utilized for the treatment of cancer, including small cell lung, cervical, breast and prostate carcinomas. Where the cause of cancer is related to a defective P25.1 gene, treatment can be administered, for example, in the form of gene replacement therapy. Specifically, one or more copies of a normal P25.1 gene or a portion of the P25.1 gene that directs the production of an P25.1 gene product exhibiting normal function, may be inserted into the appropriate cells within a patient or animal subject, using vectors that include, but are not limited to adenovirus, adeno-associated virus, retrovirus and herpes virus vectors, in addition to other particles that introduce DNA into cells, such as liposomes.

Because the P25.1 gene is expressed in the brain, possibly including the cortex, thalamus, brain stem and spinal cord and hypothalamus, such gene replacement therapy techniques should be capable of delivering P25.1 gene sequences to these cell types within patients. Thus, the techniques for delivery of the P25.1 gene sequences should be designed to readily cross the blood-brain barrier, that are well known to those of skill in the art (PCT WO89/10134), or, alternatively, should involve direct administration of such P25.1 gene sequences to the site of the cells in which the P25.1 gene sequences are to be expressed. Alternatively, targeted homologous recombination can be utilized to correct the defective endogenous P25.1 gene in the appropriate tissue; *e.g.,* brain tissue. In animals, targeted homologous recombination can be used to correct the defect in ES cells in order to generate offspring with a corrected trait.

Additional methods which may be utilized to increase the overall level of P25.1 gene expression and/or P25.1 activity include the introduction of appropriate P25.1-expressing cells, preferably autologous cells, into a patient at positions and in numbers which are sufficient to ameliorate the progression of cancer. Such cells may be either recombinant or non-recombinant. Among the cells which can be administered to increase the overall level of P25.1 gene expression in a patient are normal cells, or *e.g.*, hypothalamus cells, that express the P25.1 gene. The cells can be administered at the anatomical site in the brain, or as part of a tissue graft located at a different site in the body. Such cell-based gene therapy techniques are well known to those skilled in the art (Anderson et al., U.S. Pat. No. 5,399,349; Mulligan & Wilson, U.S. Pat. No. 5,460,959).

Finally, compounds, identified in the assays described above, that stimulate or enhance the signal transduced by activated or bound P25.1, e.g., by activating downstream signaling proteins in the P25.1 signal transduction pathway and thereby by-passing the defective P25.1, can be used to treat cancer or modulate NE differentiation. The formulation and mode of administration will depend upon the physico-chemical properties of the compound. The administration should include known techniques that allow for a crossing of the blood-brain barrier.

### Inhibition of P25.1 Expression

In an alternate embodiment, cancer therapy can be designed to reduce the level of endogenous P25.1 gene expression, *e.g.,* using antisense or ribozyme approaches to inhibit or prevent translation of P25.1 mRNA transcripts; triple helix approaches to inhibit transcription of the P25.1 gene; or targeted homologous recombination to inactivate or "knock out"the P25.1 gene or its endogenous promoter. Such gene therapy may be utilized for treatment of cancer, such as small cell lung, cervical, breast and prostate carcinomas, where the inhibition of P25.1 expression is designed to prevent NE differentiation. Because the P25.1 gene is expressed in the brain, in addition to many other tissues, delivery techniques should be preferably designed to cross the blood-brain barrier (PCT WO89/10134). Alternatively, the antisense, ribozyme or DNA constructs described herein could be administered directly to the site containing the target cells.

Antisense approaches involve the design of oligonucleotides (either DNA or RNA) that are complementary to mRNA. The antisense oligonucleotides will bind to the complementary mRNA transcripts and prevent translation. Absolute complementarily, although preferred, is not required. A sequence "complementary" to a portion of an RNA, as referred to herein, means a sequence having sufficient complementarily to be able to hybridize with the RNA, forming a stable duplex; in the case of double-stranded antisense nucleic acids, a single strand of the duplex DNA may thus be tested, or triplex formation may be assayed. The ability to hybridize will depend on both the degree of complementarily and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

While antisense nucleotides complementary to the coding region sequence could be used, those complementary to the transcribed, untranslated region are most preferred. Oligonucleotides that are complementary to the 5' end of the message, e.g., the 5' untranslated sequence up to and including the AUG initiation codon, should work most efficiently at inhibiting translation (*see* SEQ ID. NOS:2-16, herein). However, sequences complementary to the 3' untranslated sequences of mRNAs have recently been shown to be effective at inhibiting translation of mRNAs as well (Wagner, *Nature* 372:333-335, 1994). Thus, oligonucleotides complementary to either the 5'- or 3'-non-translated, non-coding regions of P25.1 could most preferably be used in an antisense approach to inhibit translation of endogenous P25.1 mRNA.

Oligonucleotides complementary to the 5' untranslated region of the mRNA should preferably include the complement of the AUG start codon. Antisense oligonucleotides complementary to mRNA coding regions are less efficient inhibitors of translation but could be used in accordance with the invention. Whether designed to hybridize to the 5'-, 3'- or coding region of P25.1 mRNA, antisense nucleic acids should be at least ten nucleotides in length, and are preferably oligonucleotides ranging from 10 to about 25 nucleotides in length. In specific aspects the oligonucleotide is at least 15 nucleotides, at least 17 nucleotides, or at least 21 nucleotides (*see* SEQ ID NOS:2-16).

Regardless of the choice of target sequence, it is preferred that in vitro studies are first performed to quantitate the ability of the antisense oligonucleotide to inhibit gene expression, it is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors *in vivo*)*,* or agents facilitating or modulating transport across the cell membrane (Letsinger et al., *Proc. Natl. Acad. Sci. USA* 86:6553-6556, 1989; Lemaitre et al., *Proc. Natl. Acad. Sci. USA* 84:648-652,1987; PCT WO88/09810) or the blood-brain barrier (PCT WO89/10134), or the nuclear membrane, and may include hybridization-triggered cleavage agents (Krol et al., *BioTechniques* 6:958-976, 1988) or intercalating agents. (Zon, *Pharm. Res.* 5:539-549, 1988). To this end, the oligonucleotide may be conjugated to another molecule, *e.g.,* a peptide, hybridization triggered cross-linking agent, transport agent, hybridization-triggered cleavage agent, etc.

The antisense oligonucleotide may comprise at least one modified base moiety which is selected from the group including but not limited to 5- fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. The antisense oligonucleotide may also comprise at least one modified sugar moiety selected from the group including but not limited to arabinose, 2 fluoroarabinose, xylulose, and hexose.

In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof. In yet another embodiment, the antisense oligonucleotide is an alpha-anomeric oligonucleotide. An alpha-anomeric oligonucleotide forms specific double-stranded hybrids with complementary RNA in which, contrary to the usual beta-units, the strands run parallel to each other (Gautier et al., *Nucl. Acids Res.* 15:6625-6641, 1987). The oligonucleotide is a 2'-O-methylribonucleotide (Inoue et al., *Nucl. Acids Res.* 15:6131-6148, 1987), or a chimeric RNA DNA analogue (Inoue et al., *FEBS Lett.* 215: 327-330, 1987).

Oligonucleotides of the invention may be synthesized by standard methods known in the art, *e.g.,* by use of an automated DNA synthesizer (such as are commercially available from Biosearch, Applied Biosystems, etc.). As examples, phosphorothioate oligonucleotides may be synthesized by the method of Stein et al. *(Nucl. Acids Res.* 16:3209, 1988), methylphosphonate oligonucleotides can be prepared by use of controlled pore glass polymer supports (Sarin et al., *Proc. Natl. Acad. Sci. USA* 85:7448-7451, 1988), etc. The antisense molecules should be delivered to cells that express the P25.1 *in vivo,* e.g., cancer or other cells and tissues. A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense sufficient to suppress translation of endogenous mRNAs. Therefore, a preferred approach utilizes a recombinant DNA construct in which the antisense oligonucleotide is placed under the control of a strong *pol* III or *pol* II promoter. The use of such a construct to transfect target cells in the patient will result in the transcription of sufficient amounts of single stranded RNAs that will form complementary base pairs with the endogenous P25.1 transcripts and thereby prevent translation of the P25.1 mRNA. For example, a vector can be introduced *in vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, *Nature* 290:304-310, 1981), the promoter contained in the 3' long terminal repeat of Rous sarcoma virus (Yamamoto et al., *Cell* 22:787-797, 1980), the herpes thymidine kinase promoter (Wagner et al., *Proc. Natl. Acad. Sci. USA* 78:1441-1445,1981), the regulatory sequences of the metallothionein gene (Brinster et al., *Nature* 296:39-42, 1982). Any type of plasmid, cosmid, YAC or viral vector can be used to prepare the recombinant DNA construct that can be introduced directly into the tissue site; *e.g*., the tumor or other tissues. Alternatively, viral vectors can be used which selectively infect the desired tissue; *(e.g.,* for brain, herpesvirus vectors may be used), in which case administration may be accomplished by another route (e.g., systemically).

Ribozyme molecules designed to catalytically cleave P25.1 mRNA transcripts can also be used to prevent translation of P25.1 mRNA and expression of P25.1. (PCT WO90/1136 Sarver et al., *Science* 247:1222-1225, 1990). While ribozymes that cleave mRNA at site specific recognition sequences can be used to destroy P25.1 mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, *Nature,* 334:585-591, 1988. There are numerous potential hammerhead ribozyme cleavage sites within the nucleotide sequence of human P25.1 cDNA (see FIG. 1B, and SEQ ID:]). Preferably the ribozyme is engineered so that the cleavage recognition site is located near the 5' end of the P25.1 mRNA *(i.e.,* to increase efficiency and minimize the intracellular accumulation of non-functional mRNA transcripts).

The ribozymes of the present invention also include RNA endoribonucleases (hereinafter "Cech-type ribozymes") such as the one which occurs naturally in *Tetrahymena thermophila* (known as the IVS, or L-19 IVS RNA) and which have been described by Thomas Cech and collaborators (Zaug et al., *Science* 224:574-578, 1984; Zaug and Cech, *Science* 231:470-475, 1986; Zaug et al., *Nature* 324:429-433, 1986; WO 88/04300; Been and Cech, *Cell* 47:207-216,1986). The Cech-type ribozymes have an eight base pair active site that hybridizes to a target RNA sequence where after cleavage of the target RNA takes place. The invention encompasses those Cech-type ribozymes that target eight base-pair active site sequences that are present in P25.1.

As in the antisense approach, the ribozymes can be composed of modified oligonucleotides *(e,g.,* for improved stability, targeting, etc.) and should be delivered to cells which express the P25.1 *in vivo, e,g.,* prostate, brain, liver, etc. A preferred method of delivery involves using a DNA construct "encoding" the ribozyme under the control of a strong constitutive *pol* III or *pol* II promoter, so that transfected cells will produce sufficient quantities of the ribozyme to destroy endogenous P25.1 messages and inhibit translation. Because ribozymes, unlike antisense oligonucleotides, are catalytic, a lower intracellular concentration is required for efficiency. Endogenous P25.1 gene expression can also be reduced by inactivating or "knocking out" the P25.1 gene or its promoter using targeted homologous recombination (Smithies et al., *Nature* 317:230-234, 1985; Thomas & Capecchi, *Cell* 51:503-512, 1987; Thompson et al., *Cell* 5:313-321,1989)*.*

For example, a mutant, non-functional P25.1 (or a completely unrelated DNA sequence) flanked by DNA homologous to the endogenous P25.1 gene can be used, with or without a selectable marker and/or a negative selectable marker, to transfect cells that express P25.1 *in vivo.* Insertion of the DNA construct, via targeted homologous recombination, results in inactivation of the P25.1 gene. Such approaches are particularly suited in the agricultural field where modifications to ES (embryonic stem) cells can be used to generate animal offspring with an inactive P25.1. However this approach can be adapted for use in humans provided the recombinant DNA constructs are directly administered or targeted to the required site *in vivo* using appropriate viral vectors, *e.g.,* herpes virus vectors for delivery to brain tissue; e.g., the hypothalamus and/or choroid plexus.

Alternatively, endogenous P25.1 gene expression can be reduced by targeting deoxyribonucleotide sequences complementary to the regulatory region of the P25.1 gene *(i.e.,* the P25.1 promoter and/or enhancers) to form triple helical structures that prevent transcription of the P25.1 gene in target cells in the body. (Helen, *Anticancer Drug Des.,* 6:569-84,1991; Helene et al*., Ann, N.Y. Acad. Sci.,* 660:27-36, 1992; and Maher, *Bioassays* 14:807-15, 1992).

### Screening Assays for Drugs Useful in Cancer Treatment

At least three different assay systems can be designed and used to identify compounds or compositions that modulate P25.1 activity or P25.1 gene expression, and therefore, modulate NE differentiation and/or cancer. The systems described below may be formulated into kits. To this end, the P25.1 or cells expressing the P25.1 can be packaged in a variety of containers, *e.g.,* vials, tubes, microtitre well plates, bottles, and the like. Other reagents can be included in separate containers and provided with the kit; e.g., positive controls samples, negative control samples, P25.1 peptides, buffers, cell culture media, etc.

***Cell based assays.*** In accordance with the invention, a cell-based assay system can be used to screen for compounds that modulate the activity of the P25.1 to identify cancer for the treatment of cancer. To this end, cells that endogenously express P25.1 and/or IGFBP-rP1 can be used to screen for compounds. Alternatively, cell lines, such as 293 cells, COS cells, CHO cells, fibroblasts, and the like, genetically engineered to express P25.1 and/or IGFBP-rP1 can be used for screening purposes. Preferably, host cells genetically engineered to express a functional IGFBP-rP1 that responds to activation by P25.1 peptides can be used as an endpoint in the assay; e.g., as measured by a chemical, physiological, biological, or phenotypic change, induction of a host cell gene or a reporter gene, change in cAMP levels, adenylyl cyclase activity, host cell G protein activity, extracellular acidification rate, host cell kinase activity, proliferation, differentiation, etc.

To be useful in screening assays, the host cells expressing functional P25.1 and/or IGFBP-rP1 should give a significant response to P25.1, preferably greater than 5-fold induction over background. Host cells should preferably possess a number of characteristics, depending on the readout, to maximize the inductive response by P25.1 peptides.

For example, in a CRE reporter gene system, for detecting a strong induction of a CRE reporter gene: (a) a low natural level of cAMP, (b) a high level of adenylyl cyclase, (c) a high level of protein kinase A, (d) a low level of phosphodiesterases, and (e) a high level of cAMP response element binding protein would be advantageous. To increase response to,*e.g*, a P25.1 peptide linked to such a system, host cells could be engineered to express a greater amount of favorable factors or a lesser amount of unfavorable factors. In addition, alternative pathways for induction of the CRE reporter could be eliminated to reduce basal levels.

In utilizing such cell systems, the cells expressing the P25.1 and/or IGFBP-rP1 are exposed to a test compound or controls. After exposure, the cells can be assayed to measure NE cellular differentiation or alternatively, the expression and/or activity of components of the signal transduction pathway of P25.1 and/or IGFBP-rP1, or the activity of the signal transduction pathway itself can be assayed. For example, after exposure, cell lysates can be assayed for induction of cAMP, or modulation of Ras, PKA, RAP1, B-Raf, Mek, or MAPK. In screening for compounds that may act as antagonists of P25.1, it may be advantageous to over-express IGFBP-rP1 to test for inhibition of signal transduction by the test compound as compared to controls. P25.1 contains a nuclear translocation signal (FIG. 1B), and has been shown herein, to undergo intracellular nuclear transport. In another embodiment of the invention, test compounds are selected based on their ability to regulate nuclear translocation of P25.1 and/or IGFBP-rP1.

***Non-cell based assays.*** In addition to cell based assays, non-cell based assay systems may be used to identify compounds that interact with, *e.g.,* bind to P25.1. Such compounds may act as antagonists or agonists of P25.1 activity and may be used in the treatment of cancer. Soluble P25.1 may be recombinantly expressed and utilized in non-cell based assays to identify compounds that bind to P25.1. Recombinantly expressed P25.1 polypeptides or fusion proteins can be used in the non-cell based screening assays. Alternatively, peptides corresponding to one or more P25.1 domains, or fusion proteins containing one or more of the P25.1 domains can be used in non-cell based assay systems to identify compounds that bind to P25.1; such compounds may be useful to modulate the signal transduction pathway of P25.1. In non-cell based assays the recombinantly expressed P25.1 may be attached to a solid substrate such as a test tube, microtitre well or a column, by means well known to those in the art. The test compounds are then assayed for their ability to bind to the P25.1.

In one aspect of the invention the screens may be designed to identify compounds that antagonize the interaction between P25.1 and P25.1 ligands such as IGFBP-rP1. In such screens, the P25.1 ligands *(i.e.,* IGFBP-rP1) are labeled and test compounds can be assayed for their ability to antagonize the binding of labeled ligand to P25.1.

***Assays for compounds or compositions that modulate expression of the P25.** In vitro* cell based assays may be designed to screen for compounds that regulate P25.1 expression at either the transcriptional or translational level. To identify compounds that regulate P25.1 translation, cells or *in vitro* cell lysates containing P25.1 transcripts may be tested for modulation of P25.1 mRNA translation. To assay for inhibitors of P25.1 translation, test compounds are assayed for their ability to modulate the translation of P25.1 mRNA in *in vitro* translation extracts. Compounds that decrease the level of P25.1 expression, either at the transcriptional or translational level, may be useful for treatment of some forms of cancer. In contrast, those compounds that increase the expression of P25.1 may be useful for treatment of other forms of cancer.

### Compounds that can be Screened in Accordance With the Invention

The assays described above can identify compounds which affect P25.1 activity. For example, compounds that affect P25.1 activity include but are not limited to compounds that bind to the P25.1, inhibit binding of the natural ligand (IGFBP-rP1), and either activate signal transduction (agonists) or block activation (antagonists), and compounds that bind to the natural ligand of P25.1 and neutralize ligand activity. Compounds that affect P25.1 gene activity (by affecting P25.1 gene expression, including molecules, e.g., proteins or small organic molecules, that affect transcription or interfere with splicing events so that expression of the full-length or the truncated form of P25.1 can be modulated) can also be identified on the screens of the invention. However, it should be noted that the assays described can also identify compounds that modulate P25.1 signal transduction (e.g., compounds which affect downstream signaling events, such as inhibitors or enhancers of G protein activities, or of Ras, PKA, RAP1, B-Raf, Mek, or MAPK, which participate in transducing the signal activated by IGFBP-rP1 binding to the P25.1). The identification and use of such compounds that affect signaling events downstream of P25.1 and thus modulate effects of P25.1 on the cancer progression are within the scope of the invention.

The compounds which may be screened in accordance with the invention include, but are not limited to peptides, antibodies and fragments thereof, and other organic compounds (*e.g.*, peptidomimetics) that bind to P25.1 and either mimic the activity triggered by IGFBP-rP *(i.e.,* agonists) or inhibit the activity triggered by IGFBP-rP 1 (*i.e.,* antagonists); as well as peptides, antibodies or fragments thereof, and other organic compounds that include mutant or truncated P25.1 molecules (or a portion thereof) and that bind to and "neutralize" IGFBP-rP1. Compounds may include, but are not limited to, peptides such as, for example, soluble peptides, including but not limited to members of random peptide libraries; (Lam et al., *Nature* 354:82-84, 1991; Houghten et al., *Nature* 354:84-86, 1991), and combinatorial chemistry-derived molecular library made of D- and/or L-configuration amino acids, phosphopeptides (including, but not limited to, members of random or partially degenerate, directed phosphopeptide libraries; *e.g.,* Songyang et al., *Cell* 72: 67-778, 1993), antibodies (including, but not limited to, polyclonal, monoclonal, humanized, anti-idiotypic, chimeric or single chain antibodies, and FAb, F(ab')₂ and FAb expression library fragments, and epitope-binding fragments thereof), and small organic or inorganic molecules. Other compounds which can be screened in accordance with the invention include, but are not limited to, small organic molecules that are able to cross the blood-brain barrier, gain entry into an appropriate cell and affect the expression of the P25.1 gene or some other gene involved in the P25.1 signal transduction pathway (*e.g*., by interacting with the regulatory region or transcription factors involved in gene expression), or such compounds that affect the activity of P25.1 or the activity of some other intracellular factor involved in the P25.1 signal transduction pathway, such as, for example, IGFBP-rP1, Ras, PKA, RAP1, B-Raf, Mek, or MAPK.

Compounds identified via assays such as those described herein may be useful, for example, in elaborating the biological function of the P25.1 gene product, and for treating cancer. Assays for testing the efficacy of compounds identified in the cellular screen can be tested in cell-based or animal model systems. Such animal models may be used as test substrates for the identification of drugs, pharmaceuticals, therapies and interventions which may be effective in treating such disorders. For example, animal models may be exposed to a compound, suspected of exhibiting an ability to treat cancer, at a sufficient concentration and for a time sufficient to elicit such cancer treatment in the exposed animals. The response of the animals to the exposure may be monitored by assessing the reversal of cancer.

### Delivery of Soluble P25.1 Polypeptides

Genetically engineered cells that express soluble P25.1 domains or fusion proteins *e.g*., fusion Ig molecules or P25.1^{HA}, can be administered *in vivo* where they may function as 'bioreactors" that deliver a supply of the soluble molecules. Such soluble P25.1 polypeptides and fusion proteins, when expressed at appropriate concentrations, should neutralize, "mop up," or potentially activate, as the case may be, the native ligand for P25.1 (*i.e*., IGFBP-rP1) and thus act as modulators of P25.1 activity for cancer treatment purposes.

### Pharmaceutical Formulations and Methods of Treating Cancer

The invention encompasses methods and compositions for treating cancer and modulating NE differentiation. Because enhancement of P25.1 gene product function results in activation of the P25.1 pathway *(e.g.,* downstream activation) and NE differentiation, inhibition of P25.1 activity may facilitate progress in treating cancers such as small cell lung, cervical, breast and prostate carcinomas. Alternatively, progression of certain cancers may be facilitated by a lower than normal levels of P25.1 gene expression, and/or P25.1 gene activity, and/or down regulating activity of the P25.1 pathway (*e.g*., by targeting downstream signaling events). Different approaches are discussed. Agonists of P25.1 can be used to induce NE differentiation in certain cancers such as small cell lung, cervical, breast and prostate carcinomas. Antagonists of P25.1 activity can be used to inhibit NE differentiation in these same cancer types. It is not necessary that the compound demonstrate absolute specificity for the P25.1. For example, compounds that agonize both P25.1, and other unknown IGFBP-rP1 interaction molecules, could be used. Such compounds could be administered so that delivery to the prostate, or elsewhere, is optimized to achieve cancer treatment, and potential side effects may well be tolerated. Compounds that do not demonstrate a specificity for P25.1 can be administered in conjunction with another therapy or drug to control the side-effects that may result from modulating other molecules (*e.g*., other IGFBP-rPs); however, compounds which demonstrate a preference or selectivity for P25.1 are preferred.

***Dose determinations.*** Toxicity and therapeutic efficacy of such compounds can be determined by standard pharmaceutical and toxicologic procedures in cell cultures or experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀ /ED₅₀. Compounds that exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ *(i.e.,* the concentration of the test compound that achieves a half-maximal inhibition of symptoms) as determined in cell culture. Such information can be used to more accurately determine useful doses in humans. Levels in plasma may be measured, for example, by high performance liquid chromatography.

***Formulations and use.*** Pharmaceutical compositions for use in accordance with the present invention may be formulated in a conventional manner using one or more physiologically acceptable carriers or excipients. Thus, the compounds and their physiologically acceptable salts and solvates may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (*e.g*., pre-gelatinized maize starch, polyvinylpyrrolidone or hydroxypropyl methylcellulose); fillers (*e.g*., lactose, microcrystalline cellulose or calcium hydrogen phosphate); lubricants *(e.g.,* magnesium stearate, talc or silica); disintegrants *(e.g.,* potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g.*, almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For buccal administration the compositions may take the form of tablets or lozenges formulated in conventional manner.

For administration by inhalation, the compounds for use according to the present invention are conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of gelatin for use in an inhaler or insufflator may be formulated containing a powder mix of the compound and a suitable powder base such as lactose or starch.

The compounds may be formulated for parenteral administration by injection, *e.g*., by bolus injection or continuous infusion. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulary agents such as suspending, stabilizing and/or dispersing agents. Alternatively, the active ingredient may be in powder form for constitution with a suitable vehicle, *e.g.,* sterile pyrogen-free water, before use. The compounds may also be formulated in rectal compositions such as suppositories or retention enemas, *e.g*., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device that may contain one or more unit dosage forms containing the active ingredient. The pack may, for example, comprise metal or plastic foil, such as a blister pack. The pack or dispenser device may be accompanied by instructions for administration.

### EXAMPLE 1

### Cloning of a cDNA for a Novel IGFBP-rP1 Interacting Protein, P25.1

The following example describes the cloning and isolation of P25.1, a cDNA encoding a novel IGFBP-rP1 interacting protein. The results show the discovery of a novel cDNA, 913 bp in length, encoding a novel 222 amino acid protein that contains a nuclear localization signal and a possible myristilation site.

***Isolation of Novel cDNA Clone using The Yeast-Two-Hybrid System.*** A schematic diagram of the Yeast-two-hybrid method used to isolate the IGFBP-rP1 interacting protein, P25.1, is show in Figure 1. Briefly, five µg of total RNA from MCF-7 breast cancer cells was used to make a cDNA library employing the ''Match Maker" Yeast-Two-Hybrid cDNA construction Kit (Clontech). This library was used, along with the full length IGFBP-rP1 cDNA fused to the GAL4 DNA binding domain, with the Match Maker Yeast-Two-Hybrid screening kit to isolate eighty-eight positive colonies on selective media. Sixty-five colonies remained positive when re-screened for β-galactosidase activity. Upon sequencing, it was discovered that all sixty-five colonies contained the same novel cDNA of approximately 650bp in length; now called 25.1. The full-length cDNA clone was then isolated using a "5' RACE rapid amplification of 5' kit" (Life Technologies) and a gene-specific primer for 25.1 (i.e., priming from a position 350bp downstream of what we now know is the *bona fide* 5' end on the full-length cDNA). Standard restriction enzyme digestion and subcloning technology was used to isolate the full-length cDNA clone.

***Discovery of a Novel cDNA**.* The cDNA clone was sequenced to reveal a 913 bp cDNA with an open reading frame coding for 222 amino acids, with a putative nuclear localization signal and a possible single myristylation site (Figure 2, and SEQ ID NO:1). A ''BLAST'' search of known data bases through the National Center of Biotechnology Institute ("NCBI") revealed that the sequence was novel (not in GenBank), and (until the present invention) of unknown function.

### EXAMPLE 2

### Expression Pattern of Multiple Species of P25.1 mRNA in Human Tissues

The following example describes the P25.1 mRNA expression pattern in a variety of human tissue samples. The results show the discovery of three novel species of P25.1 mRNA, that are differentially regulated within, and among the various tissues, and suggest that P25.1 mRNA expression levels may be a useful diagnostic marker for cancer progression.

***Human Multiple-Tissue Northern Blot**.* A human multi-tissue Northern blot (mRNA blot) was purchased from Clontech that contained 2 µg per sample lane of mRNA from each of the indicated tissues (spleen, thymus, prostate, testis, small intestine, colon, peripheral blood leukocytes, heart, brain, placenta, lung, liver, skeletal muscle, kidney, and pancreas). The 25.1 cDNA was used to probe Northern blot, and was radiolabeled with [³²P]-α-dCTP utilizing a random DNA priming kit, "Prime It" (Stratagene). The blot was hybridized using 20 million cpm in 10 milliliters of Rapid Hybridization Solution (Amersham-Pharmacia Biotech, Inc.) at 65°C overnight. The blot was washed using standard procedures, as is well known in the art, and as suggested by the manufacture.

***Discovery of Three Differentially Regulated P25.1 Transcripts**.* Hybridization of a human multi-tissue Northern blot with radiolabeled 25.1 cDNA probe revealed ubiquitous expression of three differentially regulated transcripts having sizes of 1.3, 3.4 and 8.5kb (Figure 3). The expression level of the three mRNA species varies both within and among the various human tissue samples. This discovery indicates that the three P25.1 mRNA species are ubiquitously expressed but differentially regulated in normal human tissues.

It was further discovered (data not shown) that mRNA levels of the three transcripts in cancer cells were generally reduced relative to the levels in normal tissues indicating that P25.1 mRNA is a useful diagnostic marker for assessing malignant progression.

### EXAMPLE 3

### Confirmation of IGFBP-rP1 and P25.1 Interaction

The following example describes construction and production of in-frame fusion proteins of P25.1 with the bacterial protein GST, and with the HA epitope tag. This example also describes production of an antibody molecule capable of forming specific associations with P25.1, and further describes western immunoblots, using affinity purified antibody, of breast and prostate cancer cell lines showing endogenous expression of P25.1 protein. Finally, the results of this example show the discovery of an association between P25.1 and IGFBP-rP1, and provide confirmation of the original Yeast-two-hybrid cloning strategy.

***Production of an Antibody Against P25.1***^{***HA***} ***Fusion Protein.*** The antibody production process is shown schematically in Figure 5. Briefly, the cDNA of P25.1 was epitope-tagged (at the C-terminus of P25.1; after nucleotide 714 of the cDNA) with the nine amino acid HA epitope (Tyr Pro Tyr Asp Val Pro Asp Tyr Ala) using PCR, and named P25.1^{HA}. P25.1^{HA} was then fused in frame at the 5' end with DNA encoding the opening reading frame of the GST bacterial protein, that included a thrombin protease site at the fusion junction, to produce a cDNA encoding a P25.1^{HA} fusion protein ("tagged fusion protein"). After growth and induction in BL21 *E. coli* cells, a cell lysate was prepared by sonication in STE buffer, the proteins were solubilized with non-ionic detergent and a reducing agent, DTT was added. The tagged fusion protein was then bound to Glutathione Sepharose, washed with STE buffer and the P25.1 ^{HA} -fused peptide was released by digestion with thrombin protease. Three New Zealand White female rabbits were subcutaneously injected with 100 µg/rabbit of purified protein that had been mixed with an equal volume of Freund's complete adjuvant (pre-immune sera was collected 5 days before immunization). At one-month intervals, two additional injections were preformed with the same concentration of protein and Freund's adjuvant. The rabbits were exsanguinated, and sera were collected from whole blood. The titer of the antibody was tested against the antigen and conditioned media from COS-7 mammalian cells transiently expressing P25.1^{HA}. The polyclonal antibody of highest titer was subsequently purified over an affinity column made with the *E. coli*-purified P25.1^{HA} protein. As shown below in this example and specification, the purified antibody was successfully used in Western immunoblotting; immunoprecipitations, and immunocytochemistry.

***Western immuno-blotting of cell lysates from Breast and Prostate cancer cell lines.*** Equal protein quantities of cell lysates, prepared in RIPA (150 mM NaCl, 20mM Hepes pH 7.5, 1 % Triton-100, 1 % sodium deoxycholate, 0.1 % SDS, with protease inhibitor cocktail), were subjected to SDS:PAGE under reducing conditions, and transferred to nitrocellulose in Towbin Transfer Buffer. The resulting nitrocellulose blots were then blocked with 5% powdered dry milk in TBS-T. Primary antibody incubations of the blots were performed in the same buffer, at the dilutions indicated, overnight at 4°C. Blots were rinsed three times in TBS-T, and the appropriate secondary antibodies were added at the indicated dilutions in TBS-T, and incubated for 1-2 hours at room temperature.

***Crosslinking ofIGFBP rP1FLAG and P25.1***^{***HA***} ***fusion peptide.*** A volume of 100 µl of conditioned medium from Hs578T breast cancer cells, 100 µl of conditioned medium from IGFBP-rP1FLAG transiently expressed in COS-7 cells, or 300 µg of IGFBP-rP1^{FLAG}, purified from a baculovirus recombinant, was mixed with either 100 µl of PBS or with 1 µg of purified P25.1^{HA} from the GST fusion in 100 µl PBS, and allowed to incubate at room temperature for 3 hours or overnight at 4°C. The interacting proteins were chemically cross linked using 10 mM DSS for 15 minutes at 4°C. The reactions were quenched in 40 mM Tris-Cl pH 7.5 and 4 mM EDTA, resuspended in non-reducing SDS:PAGE loading buffer, resolved by 12% SDS:PAGE, and transferred to nitrocellulose before blotting with a polyclonal antibody to IGFBP-rP1. The immunoreactive proteins were detected using an HRP (horseradish peroxidase) conjugated anti-rabbit secondary antibody followed by detection of Chemilluminescence.

***Co-imniunoprecipitation of IGFBP-rP1***^{***FLAG***} ***and P25.1***^{***HA***}***.*** COS-7 cells were transiently transfected using pcDNA3 plasmids containing nothing, P25.1^{HA}, IGFBP-rP1^{FLAG}, or with both plasmids containing P25.1^{HA}and IGFBP-rP1^{FLAG}, along with Fugene 6 reagent (Roche Laboratories). All plasmid concentrations were equalized with pcDNA3. Three days after transfection, the cells were collected, washed once with PBS, resuspended in PBS and lysed by sonication. After removing insoluble material by centrifugation, the cell lysates were first incubated with either α-P25.1 or α-IGFBP-rP1 polyclonal antibodies that had been pre-incubated with Protein A Sepharose (Amersham-Pharmacia). The immunoprecipitated complexes were pelleted by centrifugation and washed three times in PBS. The protein complexes were then eluted (dissolved) by boiling in reducing SDS:PAGE sample buffer, subjected to 12% SDS:PAGE, and transferred to nitrocellulose using Towbin Transfer Buffer. After blocking the nitrocellulose with 5% powdered dry milk in TBS-T (Tris-buffered saline with Tween 20), the second monoclonal antibody, corresponding to the epitope tag of the corresponding co-immunoprecipitated protein, was used for immunoblotting, and finally detected with HRP conjugated anti-mouse "secondary" antibodies and chemilluminescence.

***Mixing and Co-immunoprecipitations.*** A mixing experiment was used to verify the interaction between the two proteins using 3-day conditioned media from Hs578T or MCF-7 breast cancer cells, transiently expressing vectors encoding, P25.1^{HA}, IGFBP-rP1^{FLAG}, or nothing. Equal volumes of the indicated conditioned media were incubated overnight at 4°C. Co-immunoprecipitation and Western immunoblotting of the protein complexes was performed as described above.

***Immunocytochemical Studies of Endogenous P25.1 and IGFBP-rP1.*** Endogenously expressed P25.1 and IGFBP-rP1 proteins were examined using immunofluorescence with either α-II25.1 Ab or α-IGFBP-rP1 Ab in the prostate epithelial cancer cell line, M12.

***Discovery of Reduced Expression of Endogenous P25.1 in Cancer Cells.*** The affinity purified α-P25.1 antibody (Figure 5) was used to probe a Western immunoblot containing samples of cellular lysates from breast and prostate cancer cell lines: HMEC (primary mammary epithelial cells), MCF-7, Hs578T breast cancer cells, M12-pcDNA, and M12-rP1 stable prostate cancer cell lines (Figure 6). The results show that P25.1 is expressed at low levels in breast and prostate cancer cell lines, relative to the level of expression seen in primary mammary epithelial cells, showing that P25.1 expression is down-regulated in cancer cells.

***Studies Confirming the Interaction Between IGFBP-rP1 and P25.1.* Chemical** cross-linking, co-immunoprecipitations, and mixing experiments were used to show physical interaction of both recombinant and endogenous IGFBP-rP1 with transiently expressed or recombinant P25.1^{HA} in various systems. Endogenous IGFBP-rP 1 from breast cancer cells, as well as transient and recombinant forms were able to interact with various sources of P25.1. This interaction was shown *in vitro* by solution binding of the two peptides and either chemically crosslinking (Figure 7), or immunoprecipitation (Figure 8) of the complex and then verification by Western immuno-blotting (Figures 2C and 2D). Additionally, the two proteins associated, *in vivo* inside the cell as shown by co-immunoprecipitations of transient expressed P25.1 and endogenous or transient expressed IGFBP-rP1(Figure 2E).

***Discovery of Nuclear Translocation of Endogenous P25.1 and IGFBP-rP1.*** Immunofluorescence of endogenous P25.1 and IGFBP-rP1 on cells that had been synchronized and then allowed to replicate, showed translocation and co-localization between the cytosol and nucleus in a cell cycle dependent manner.

### EXAMPLE 4

### Investigation of the Biological Role of P25.1 in Relation to IGFBP-rP

The following example describes the result of transiently expressing P25.1 in M12-IGFBP-rP1 cells, which express IGFBP-rP1 in a stable, constitutive manner. Immunocytochemical characterization revealed that transient P25.1 expression induced neuroendocrine differentiation.

***Transient Transfections.*** pcDNA3-based plasmids with the indicated cDNA were transiently transfected into various mammalian cells using the Fugene 6 reagent according to the manufactures' suggestions. Briefly, 100 mm dishes with cell densities of 60-70% were transfected with 10 µg of the indicated plasmid which had been precipitated with 20 µl of Fugene 6 reagent (15 minutes at room temperature). Plasmid concentrations, in the case of single or dual transfections, were standardized with control vector, pcDNA3. For neuroendocrine differentiation studies, the transfections were done in 6-well dishes with up to 8 µg/well of plasmid DNA for 6-18 hours, and then trypsinized and seeded into new dishes for the subsequent experiments.

***Immunofluorescence.*** Cells were grown in 24-well dishes for the indicated times, plus or minus transfection, and/or treatment. The cells were fixed to the well in 4% paraformaldehyde for 10 minutes at room temperature, and then permeabilized in 50% methanol/50% acetone for 1-2 minutes at room temperature. The cells were blocked in 0.25% normal goat serum in PBS + 0.1% Triton-X 100. Primary antibody incubations were done at the indicated dilutions in the same buffer for 1-2 hours at room temperature, or if necessary overnight at 4°C. Each well was rinsed three times in PBS and fluorescent-conjugated secondary antibodies were added along with Hoescht's stain (1 µg/ml) for 1-2 hours at room temperature. Each well was washed three times extensively with PBS and then viewed under a Nikon inverted phase, fluorescent microscope with an analog camera.

***Discovery that Transient Expression of P25.1***^{***HA***} ***Enhances Neuroendocrine Differentiation** in **M12-IGFBP-rP1 Cells.*** To investigate the biological role of P25.1 in relation to IGFBP-rP1, a prostate epithelial cancer cell line, M12, that stably expresses IGFBP-rP1, and is referred to as the M12-IGFBP-rP1 cell line was used. In comparison to wild-type M12 cells, the stable transfected M12-IGFBP-rP1 cells acquire an elongated morphology with multiple projections. When grown in defined media they display a suppressed growth rate compared to that of the parental cell line (control), the stable M12-pcDNA cell line. The morphological and biological changes in the M12:IGFBP-rP1 cell line could be attributed to an interaction of endogenous IGFBP-rP1 with endogenous P25.1. To investigate this possibility, P25.1^{HA} was transiently expressed in M12-IGFBP-rP1cells.

Enhanced neuroendocrine differentiation of the M12-IGFBP-rP1 cells occurred, wherein long synaptic-like dendrites developed in cells transiently expressing P25.1^{HA} (Figure 9). Additionally, these cells look neuritic in appearance, and seem to be forming pseudo-synaptic junctions with other cells that are over-expressing the IGFBP-rP1 interacting protein, P25.1. The P25.1 protein is present throughout the well differentiated, neuritic cells, and even in the proposed secretory granules of the dendritic arms. Monoclonal antibodies against neuroendorcrine markers, Chr A (Chromogranin A) and NSE (Neuron-specific enolase), were used to verify that these differentiated cells were indeed neuroendocrine cells (NE) (Figure 10).

### EXAMPLE 5

### Characterization of the Relationship Between cAMP and P25.1 in Neuroendocrine Differentiation

***8-bromo-cAMP*/*Forskolin Treatments.*** Cells were treated with 0.1 to 1 mM 8br-cAMP, diluted from a 100 mM stock, diluted in growth media as indicated. 10 µM Forskolin, diluted from a 10mM stock in growth media, was used as indicated.

***Discovery of cAMP induced regulation of P25.1.*** Treatment of M12:IGFBP-rP1 cells with the cAMP analog, 8-bromo-cAMP ("8br-cAMP"), or the agonist forskolin, in the absence of transient P25.1 expression, caused morphological changes similar to NE differentiation. Immunofluorescence of endogenous P25.1, depicted a translocation of the protein from the cytosol, in the untreated M12:IGFBP-rP1 cells, to the secretory granules prevalent in NE cells.

8br-cAMP was used on M12-IGFBP-rP1 cells transiently expressing P25.1 to determine whether cAMP-induced differentiation is related to expression of P25.1. Incubation with 8br-cAMP resulted in an increase in the number of cells transiently expressing P25.1, leading to more NE differentiated cells which stained positive with NE specific antibodies (Figure 10). Western immunoblotting of total cell lysates from the transfected cells, revealed that expression levels of P25.1 were greatly enhanced by treatment with 8br-cAMP, implying a cAMP induced regulation of P25.1 (Figure 10).

### In Summary

In fibroblasts, smooth muscle cells and adipocytes, an increase in cellular cAMP inhibits growth by suppression of the MAP kinase pathway through members of the Ras protein family (*see* "Background," above). In pre-neuronal cells, such as PC12, derived from rat adrenal medullary, activation of PKA results in growth arrest and differentiation through the Rap 1/B-Raf/Mek/MAPK pathway, and addition of nerve growth factor (NGF) augments this morphological process (*Id*)*.*

In the foregoing experiments, the M12-IGFBP-rP1 cells exhibited a suppressed growth rate in defined media, implying that the Ras > MAPK pathway may be hindered by over-expression of the IGFBP-rP 1 secretory protein. This may be explained by an interaction between IGFBP-rP1 and a limited amount of endogenous P25.1 resulting in growth suppression and partial activation of the differentiation pathway. This is further enhanced by addition of cAMP analogs or agonist which in turn activate the small G protein, Rap 1 leading to activation of B-Raf and further differentiation through the MAPK pathway. Transient expression of P25.1, a specific player in this differentiation pathway, further amplifies terminal NE differentiation in cancer cells.

## Claims

1. Cancer diagnostic assay for determining a progressive cancer *in vitro,* comprising:
(a) obtaining a sample of a tumor tissue; and
(b) performing a sequence identity assay to determine an amount of protein sequences expressed by the cDNA sequence described in SEQ ID NO:1 or expressed by a sequence having at least 90% homology with the coding region of SEQ ID NO:1, present in the sample, whereby the amount of the said protein sequences is correlated with progressive cancer.

2. Cancer diagnostic assay of claim 1, wherein the sequence identity assay is selected from the group consisting of PCR assays, ELISA immunologic assays, hybridization assays, and combinations thereof.

3. *In vitro* method for identifying test compounds having cancer therapeutic activity, comprising:
(a) contacting a test compound with a cell expressing both a IGFBP-rP1 polypeptide, and a IGFBP-rP1 interacting protein being expressed by the cDNA sequence described in SEQ ID NO:1, or expressed by a sequence having at least 90% homology with the coding region of SEQ ID NO:1, wherein either the IGFBP-rP1, or the IGFBP-rP1 interacting protein, are recombinantly expressed; and
(b) determining whether the test compound alters growth rate or differentiation of the cell, as determined by the presence or absence of specific markers of cellular differentiation, whereby test compounds that alter the growth rate or differentiation of the cell are identified as cancer therapeutic compounds.

4. *In vitro* method for identifying test compounds having cancer therapeutic activity, comprising:
(a) contacting a functional IGFBP-rP1 in the presence and absence of a test compound with a functional IGFBP-rP1 interacting protein according to SEQ ID NO:1, and determining whether the test compound inhibits the interaction between the IGFBP-rP1 interacting protein and the IGFBP-rP1;
(b) further contacting the test compounds of (a), that inhibit said interaction, with a cell expressing both a IGFBP-rP1 polypeptide, and a IGFBP-rP1 interacting protein, wherein either the IGFBP-rP1, or the IGFBP-rP1 interacting protein, are recombinantly expressed; and
(c) determining whether the test compound alters growth rate or differentiation of the cell, as determined by the presence or absence of specific markers of cellular differentiation, whereby test compounds that alter the growth rate or differentiation of the cell are identified as cancer therapeutic compounds.

5. *In vitro* method for identifying test compounds having cancer therapeutic activity, comprising:
(a) contacting a test compound with a cell expressing both a IGFBP-rP1 polypeptide, and a IGFBP-rP1 interacting protein being expressed by the cDNA sequence described in SEQ ID NO:1, or expressed by a sequence having at least 90% homology with the coding region of SEQ ID NO:1; and
(b) measuring the extent of nuclear translocation of either the IGFBP-rP1 interacting protein, or the IGFBP-rP1 polypeptide, whereby test compounds that alter the extent of nuclear translocation of either the IGFBP-rP1 interacting protein, or the IGFBP-rP1 are identified as cancer therapeutic compounds.

6. *In vitro* method for identifying test compounds having cancer therapeutic activity, comprising:
(a) contacting a test compound with a cell expressing both a IGFBP-rP1 polypeptide, and a IGFBP-rP1 interacting protein being expressed by the cDNA sequence described in SEQ ID NO:1, or expressed by a sequence having at least 90% homology with the coding region of SEQ ID NO:1; and
(b) measuring phosphorylation or activation status of an intracellular protein, wherein the intracellular protein is Ras, PKA, RAP1, B-Raf, Mek, or MAPK, whereby test compounds that alter the phosphorylation or the activation status of a said intracellular protein are identified as cancer therapeutic compounds.

7. *In vitro* method for identifying test compounds having cancer therapeutic activity, comprising:
(a) contacting a test compound with a functional IGFBP-rP1 interacting protein according to SEQ ID NO:1, and determining whether the test compound interacts with the functional IGFBP-rP1 interacting protein;
(b) further contacting the test compounds of (a), that interact with the functional IGFBP-rP1 interacting protein, with a cell expressing both a IGFBP-rP1 polypeptide, and a IGFBP-rP1 interacting protein being expressed by the cDNA sequence described in SEQ ID NO:1, or expressed by a sequence having at least 90% homology with the coding region of SEQ ID NO:1, wherein either the IGFBP-rP1, or the IGFBP-rP1 interacting protein, are recombinantly expressed; and
(c) determining whether the test compound alters growth rate or differentiation of the cell, as determined by the presence or absence of specific markers of cellular differentiation,
whereby test compounds that alter the growth rate or differentiation of the cell are identified as cancer therapeutic compounds.

8. *In vitro* method for identifying test compounds having cancer therapeutic activity, comprising:
(a) contacting a test compound with a cell or cell lysate containing mRNA sequences of IGFBP-rP1 interacting protein, the said protein being expressed by the cDNA sequence described in SEQ ID NO:1, or expressed by a sequence having at least 90% homology with the coding region of SEQ ID NO:1, and
(b) detecting translational inhibition of the IGFBP-rP1 interacting protein transcripts.

9. Antagonist of biological activity of the protein according to SEQ ID NO:1, wherein the antagonist is selected from the group consisting of an antibody against the said protein, an antisense molecule against the cDNA sequence described in SEQ ID NO:1 or against a sequence having at least 90% homology with the coding region of SEQ ID NO:1, and a ribozyme molecule.

10. Antagonist of claim 9 in which the antisense molecule is a specific antisense oligonucleotide of at least 10 bases complementary to the cDNA sequence SEQ ID NO:1.

11. Antagonist of claim 10 wherein the antisense oligonucleotide is a 15-25 base oligonucleotide incorporating an oligonucleotide sequence selected from the group consisting of: SEQ ID NOS:2-16; and combinations thereof.

12. Anticancer pharmaceutical composition comprising a therapeutically effective amount of an antagonist according to any of the claims 9-11, and a pharmaceutically acceptable carrier.

13. Anticancer pharmaceutical composition comprising an antibody that binds to a protein according to SEQ ID NO:1 or a binding fragment thereof, and a pharmaceutically acceptable carrier.

14. Anticancer pharmaceutical composition of claim 13 wherein the antibody is a humanized monoclonal antibody.

15. Anticancer pharmaceutical composition of any one of claims 12-14, wherein the cancer to be treated or prevented is small cell lung, cervical, breast, or prostate carcinoma.

## Patentansprüche

1. Krebs-Diagnosetest zum Bestimmen einer progressiven Krebserkrankung *in vitro,* umfassend:
(a) Bereitstellen einer Probe aus einem Tumorgewebe; und
(b) Durchführen eines Sequenz-Identitätstests, um eine in der Probe vorliegende Menge von Proteinsequenzen zu bestimmen, die durch die in SEQ ID NO: 1 beschriebene cDNA-Sequenz oder durch eine Sequenz exprimiert werden, die eine Homologie von mindestens 90 % mit der codierenden Region von SEQ ID NO: 1 aufweist, wodurch die Menge der Proteinsequenzen auf einen progressiven Krebs bezogen wird.

2. Krebs-Diagnosetest nach Anspruch 1, wobei der Sequenz-Identitätstest aus der Gruppe ausgewählt ist, die aus PCR-Tests, ELISA-Immuntests, Hybridisierungstests und Kombinationen davon besteht.

3. *In vitro*-Verfahren zum Identifizieren von Testverbindungen, die in der Krebstherapie wirksam sind, umfassend:
(a) Inkontaktbringen einer Testverbindung mit einer Zelle, die sowohl ein IGFBP-rP1-Polypeptid als auch ein IGFBP-rP1-interagierendes Protein exprimiert, das durch die in SEQ ID NO: 1 beschriebene cDNA-Sequenz oder durch eine Sequenz exprimiert wird, die eine Homologie von mindestens 90 % mit der codierenden Region von SEQ ID NO: 1 aufweist, wobei entweder das IGFBP-rP1 oder das IGFBP-rP1-interagierende Protein rekombinant exprimiert wird; und
(b) Bestimmen, ob die Testverbindung die Wachstumsrate oder die Differenzierung der Zelle verändert, wobei dies durch das Vorliegen oder die Abwesenheit von spezifischen Markern der Zelldifferenzierung bestimmt wird, wodurch Testverbindungen, welche die Wachstumsrate oder die Differenzierung der Zelle verändern, als Verbindungen für die Krebstherapie identifiziert werden.

4. *In vitro*-Verfahren zum Identifizieren von Testverbindungen, die in der Krebstherapie wirksam sind, umfassend:
(a) Inkontaktbringen eines funktionellen IGFBP-rP1 in Gegenwart und in Abwesenheit einer Testverbindung mit einem funktionellen IGFBP-rP1-interagierenden Protein gemäß SEQ ID NO: 1 und Bestimmen, ob die Testverbindung die Wechselwirkung zwischen dem IGFBP-rP1-interagierenden Protein und dem IGFBP-rP1 hemmt;
(b) weiterhin Inkontaktbringen der Testverbindungen von (a), welche die Wechselwirkung hemmen, mit einer Zelle, die sowohl ein IGFBP-rP1-Polypeptid als auch ein IGFBP-rP1-interagierendes Protein exprimiert, wobei entweder das IGFBP-rP1 oder das IGFBP-rP1-interagierende Protein rekombinant exprimiert wird; und
(c) Bestimmen, ob die Testverbindung die Wachstumsrate oder die Differenzierung der Zelle verändert, wobei dies durch das Vorliegen oder die Abwesenheit von spezifischen Markern der Zelldifferenzierung bestimmt wird, wodurch Testverbindungen, welche die Wachstumsrate oder die Differenzierung der Zelle verändern, als Verbindungen für die Krebstherapie identifiziert werden.

5. *In vitro*-Verfahren zum Identifizieren von Testverbindungen, die in der Krebstherapie wirksam sind, umfassend:
(a) Inkontaktbringen einer Testverbindung mit einer Zelle, die sowohl ein IGFBP-rP1-Polypeptid als auch ein IGFBP-rP1-interagierendes Protein exprimiert, das durch die in SEQ ID NO: 1 beschriebene cDNA-Sequenz oder durch eine Sequenz exprimiert wird, die eine Homologie von mindestens 90 % mit der codierenden Region von SEQ ID NO: 1 aufweist; und
(b) Messen des Ausmaßes der Kem-Translokation entweder des IGFBP-rP1-interagierenden Proteins oder des IGFBP-rP1-Porypeptids, wodurch Testverbindungen, welche das Ausmaß der Kem-Translokation entweder des IGFBP-rP1-interagierenden Proteins oder des IGFBP-rP1-Polypeptids verändern, als Verbindungen für die Krebstherapie identifiziert werden.

6. *In vitro*-Verfahren zum Identifizieren von Testverbindungen, die in der Krebstherapie wirksam sind, umfassend:
(a) Inkontaktbringen einer Testverbindung mit einer Zelle, die sowohl ein IGFBP-rP1-Polypeptid als auch ein IGFBP-rP1-interagierendes Protein exprimiert, das durch die in SEQ ID NO: 1 beschriebene cDNA-Sequenz oder durch eine Sequenz exprimiert wird, die eine Homologie von mindestens 90 % mit der codierenden Region von SEQ ID NO: 1 aufweist; und
(b) Messen des Phosphorylierungs- oder Aktivierungsstatus eines intrazellulären Proteins, wobei das intrazelluläre Protein Ras, PKA, RAP1, B-Raf, Mek oder MAPK ist, wodurch Testverbindungen, welche den Phosphorylierungs-oder den Aktivierungsstatus des intrazellulären Proteins verändern, als Verbindungen für die Krebstherapie identifiziert werden.

7. *In vitro*-Verfahren zum Identifizieren von Testverbindungen, die in der Krebstherapie wirksam sind, umfassend:
(a) Inkontaktbringen einer Testverbindung mit einem funktionellen IGFBP-rP1-interagierenden Protein gemäß SEQ ID NO: 1 und Bestimmen, ob die Testverbindung mit dem funktionellen IGFBP-rP1-interagierenden Protein interagiert;
(b) weiterhin Inkontaktbringen der Testverbindungen von (a), die mit dem funktionellen IGFBP-rP1-interagierenden Protein interagieren, mit einer Zelle, die sowohl ein IGFBP-rP1-Polypeptid als auch ein IGFBP-rP1-interagierendes Protein exprimiert, das durch die in SEQ ID NO: 1 beschriebene cDNA-Sequenz oder durch eine Sequenz exprimiert wird, die eine Homologie von mindestens 90 % mit der codierenden Region von SEQ ID NO: 1 aufweist, wobei entweder das IGFBP-rP1 oder das IGFBP-rP1-interagierende Protein rekombinant exprimiert wird; und
(c) Bestimmen, ob die Testverbindung die Wachstumsrate oder die Differenzierung der Zelle verändert, wobei dies durch das Vorliegen oder die Abwesenheit spezifischer Marker der Zelldifferenzierung bestimmt wird, wodurch Testverbindungen, welche die Wachstumsrate oder die Differenzierung der Zelle verändern, als Verbindungen für die Krebstherapie identifiziert werden.

8. *In vitro*-Verfahren zum Identifizieren von Testverbindungen, die in der Krebstherapie wirksam sind, umfassend:
(a) Inkontaktbringen einer Testverbindung mit einer Zelle oder einem Zelllysat, enthaltend mRNA-Sequenzen eines IGFBP-rP1-interagierenden Proteins, wobei das Protein durch die in SEQ ID NO: 1 beschriebene cDNA-Sequenz oder durch eine Sequenz exprimiert wird, die eine Homologie von mindestens 90 % mit der codierenden Region von SEQ ID NO: 1 aufweist; und
(b) Nachweisen der translationalen Hemmung der Transkripte des IGFBP-rP1-interagierenden Proteins.

9. Antagonist der biologischen Aktivität des Proteins gemäß SEQ ID NO: 1, wobei der Antagonist aus der Gruppe ausgewählt ist, die aus einem Antikörper gegen das Protein, einem Antisense-Molekül gegen die in SEQ ID NO: 1 beschriebene cDNA-Sequenz oder gegen eine Sequenz, die eine Homologie von mindestens 90 % mit der codierenden Region von SEQ ID NO: 1 aufweist, und einem Ribozym-Molekül besteht.

10. Antagonist nach Anspruch 9, wobei das Antisense-Molekül ein spezifisches Antisense-Oligonucleotid mit mindestens 10 Basen ist, das zur cDNA-Sequenz von SEQ ID NO: 1 komplementär ist.

11. Antagonist nach Anspruch 10, wobei das Antisense-Oligonucleotid ein 15- bis 25-Basen-Oligonucleotid ist, das eine Oligonucleotidsequenz enthält, die aus der Gruppe der SEQ ID NOs: 2 bis 16 und Kombinationen davon ausgewählt ist.

12. Pharmazeutische Zusammensetzung gegen Krebs, die eine therapeutisch wirksame Menge eines Antagonisten gemäß einem der Ansprüche 9 bis 11 und einen pharmazeutisch verträglichen Träger umfasst.

13. Pharmazeutische Zusammensetzung gegen Krebs, die einen Antikörper, der an ein Protein gemäß SEQ ID NO: 1 bindet, oder ein bindendes Fragment davon und einen pharmazeutisch verträglichen Träger umfasst.

14. Pharmazeutische Zusammensetzung gegen Krebs nach Anspruch 13, wobei der Antikörper ein humanisierter monoclonaler Antikörper ist.

15. Pharmazeutische Zusammensetzung gegen Krebs nach einem der Ansprüche 12 bis 14, wobei der Krebs, der behandelt oder verhindert werden soll, ein kleinzelliges Lungenkarzinom, Zervix-, Mamma- oder Prostatakarzinom ist.

## Revendications

1. Test de diagnostic de cancer pour la détection d'un cancer progressif *in vitro*, comprenant :
(a) l'obtention d'un échantillon d'un tissu tumoral ; et
(b) la réalisation d'un test d'identité de séquences afin d'établir un nombre de séquences protéiques exprimées par la séquence d'ADNc décrite à la SÉQ ID N° 1 ou exprimées par une séquence présentant au moins 90 % d'homologie par rapport à la région codante de la SÉQ ID N° 1, présentes dans l'échantillon, par lequel le nombre desdites séquences protéiques est lié à un cancer progressif.

2. Test de diagnostic de cancer de la revendication 1, dans lequel le test d'identité de séquences est choisi dans le groupe formé par des tests par PCR, de tests immunologiques par ELISA, des tests d'hybridation, et des combinaisons de ceux-ci.

3. Procédé *in vitro* d'identification de composés test possédant une activité thérapeutique contre un cancer, comprenant :
(a) la mise en contact d'un composé test avec une cellule exprimant à la fois un polypeptide de l' IGFBP-rP1, et une protéine interagissant avec l' IGFBP-rP1 étant exprimée par la séquence d'ADNc décrite à la SÉQ ID N° 1, ou exprimée par une séquence présentant au moins 90 % d'homologie par rapport à la région codante de la SÉQ ID N° 1, dans laquelle soit l'IGFBP-rP1, soit la protéine interagissant avec l'IGFBP-rP1, est exprimée par recombinaison ; et
(b) la détermination de si le composé test modifie ou non le taux de prolifération ou la différenciation de la cellule, comme l'établit la présence ou l'absence de marqueurs spécifiques de la différenciation cellulaire, de telle sorte que les composés test qui modifient le taux de prolifération ou la différenciation de la cellule sont identifiés comme composés thérapeutiques contre un cancer.

4. Procédé *in vitro* d'identification de composés test possédant une activité thérapeutique contre un cancer, comprenant :
(a) la mise en contact d'une IGFBP-rP1 fonctionnelle, en présence et en l'absence d'un composé test, avec une protéine interagissant avec l'IGFBP-rP1 fonctionnelle selon la SÉQ ID N° : 1, et la détermination de si le composé test inhibe ou non l'interaction entre la protéine interagissant avec l' IGFBP-rP1 et l' IGFBP-rP1 ;
(b) la mise en contact, en outre, des composés test de (a), qui inhibent ladite interaction, avec une cellule exprimant à la fois un polypeptide de l'IGFBP-rP1, et une protéine interagissant avec l'IGFBP-rP1, dans laquelle soit l'IGFBP-rP1, soit la protéine interagissant avec l'IGFBP-rP1, est exprimée par recombinaison ; et
(c) la détermination de si le composé test modifie ou non le taux de prolifération ou la différenciation de la cellule, comme l'indique la présence ou l'absence de marqueurs spécifiques de la différenciation cellulaire, de telle sorte que les composés test qui modifient le taux de prolifération ou la différenciation de la cellule sont identifiés comme composés thérapeutiques contre une tumeur.

5. Procédé *in vitro* d'identification de composés test possédant une activité thérapeutique contre un cancer, comprenant :
(a) la mise en contact d'un composé test avec une cellule exprimant à la fois un polypeptide de l'IGFBP-rP1, et une protéine interagissant avec l'IGFBP-rP1 étant exprimée par la séquence d'ADN-c décrite à la SÉQ ID N° 1, ou exprimée par une séquence présentant au moins 90 % d'homologie par rapport à la région codante de la SÉQ ID N° 1 ; et
(b) la mesure du degré de translocation nucléaire soit de la protéine interagissant avec l'IGFBP-rP1 soit du polypeptide de l'IGFBP-rP1, façon dont les composés test qui modifient le degré de translocation nucléaire soit de la protéine interagissant avec l'IGFBP-rP1, soit de l'IGFBP-rP1 sont identifiés comme composés thérapeutiques contre un cancer.

6. Procédé *in vitro* d'identification de composés test possédant une activité thérapeutique contre un cancer, comprenant :
(a) la mise en contact d'un composé test avec une cellule exprimant à la fois un polypeptide de l'IGFBP-rP1, et une protéine interagissant avec l'IGFBP-rP1 étant exprimée par la séquence d'ADN-c décrite à la SÉQ ID N° 1, ou exprimée par une séquence présentant au moins 90 % d'homologie par rapport à la région codante de la SÉQ ID N° 1 ; et
(b) la mesure de l'état de phosphorylation ou d'activation d'une protéine intracellulaire, où la protéine intracellulaire est Ras, PKA, RAP1, B-Raf, Mek ou MAPK, façon dont les composés test qui modifient l'état de phosphorylation ou d'activation d'une dite protéine intracellulaire sont identifiés comme composés thérapeutiques contre un cancer.

7. Procédé *in vitro* d'identification de composés test possédant une activité thérapeutique contre un cancer, comprenant :
(a) la mise en contact d'un composé test avec une protéine interagissant avec l'IGFBP-rP1 fonctionnelle selon la SÉQ ID N° : 1, et la détermination de si le composé test interagit ou non avec la protéine interagissant avec l'IGFBP-rP1 fonctionnelle ; et
(b) la mise en contact, en outre, des composés test de (a), qui interagissent avec la protéine interagissant avec l'IGFBP-rP1 fonctionnelle, avec une cellule exprimant à la fois un polypeptide de l'IGFBP-rP1 et une protéine interagissant avec l'IGFBP-rP1 qui est exprimée par la séquence d'ADN-c décrite à la SÉQ ID N° 1, ou exprimée par une séquence présentant au moins 90 % d'homologie par rapport à la région codante de la SÉQ ID N° 1, dans laquelle soit l'IGFBP-rP1, soit la protéine interagissant avec l'IGFBP-rP1, est exprimée par recombinaison ; et
(c) la détermination de si le composé test modifie ou non le taux de prolifération ou la différenciation de la cellule, comme l'indique la présence ou l'absence de marqueurs spécifiques de la différenciation cellulaire, façon dont les composés test qui modifient le taux de prolifération ou la différenciation de la cellule sont identifiés comme composés thérapeutiques contre un cancer.

8. Procédé *in vitro* d'identification de composés test possédant une activité thérapeutique contre un cancer, comprenant :
(a) la mise en contact d'un composé test avec une cellule ou un lysat cellulaire contenant des séquences d'ARN-m d'une protéine interagissant avec l'IGFBP-rP1, ladite protéine étant exprimée par la séquence d'ADN-c décrite à la SÉQ ID N° 1, ou exprimée par une séquence présentant au moins 90 % d'homologie par rapport à la région codante de la SÉQ ID N° 1, et
(b) la détection de l'inhibition de la traduction des produits de transcription en protéine interagissant avec l'IGFBP-rP1

9. Antagoniste de l'activité biologique de la protéine selon la. SÉQ ID N° 1, où l'antagoniste est choisi dans le groupe formé par un anticorps contre ladite protéine, une molécule antisens contre la séquence d'ADN-c décrite à la SÉQ ID N°1 ou contre une séquence présentant au moins 90 % d'homologie par rapport à la région codante de la SÉQ ID N° 1, et une molécule de ribozyme.

10. Antagoniste de la revendication 9 dans lequel la molécule antisens est un oligonucléotide antisens spécifique d'au moins 10 bases complémentaires par rapport à la séquence d'ADN-c SÉQ ID N° 1.

11. Antagoniste de la revendication 10 dans lequel l'oligonucléotide antisens est un oligonucléotide de 15 à 25 bases contenant une séquence oligonucléotidique choisie dans le groupe formé par les SÉQ ID N° 2 à 16 ; et des combinaisons de celles-ci.

12. Composition pharmaceutique anticancéreuse comprenant une quantité thérapeutiquement efficace d'un antagoniste selon l'une quelconque des revendications 9 à 11, et un excipient pharmaceutiquement acceptable.

13. Composition pharmaceutique anticancéreuse comprenant un anticorps qui se lie à une protéine selon la SÉQ ID N° 1 ou un fragment de liaison de celui-ci, et un excipient pharmaceutiquement acceptable.

14. Composition pharmaceutique anticancéreuse de la revendication 13 dans laquelle l'anticorps est un anticorps monoclonal humanisé.

15. Composition pharmaceutique anticancéreuse de l'une quelconque des revendications 12 à 14 dans laquelle le cancer à traiter ou à prévenir est le carcinome pulmonaire à petites cellules, le carcinome cervical, du sein, ou de la prostate.


### SEQUENCE LISTING

(1) GENERAL INFORMATION:
   (i) APPLICANTS: Oregon Health Sciences University and Elizabeth M. Wilson, Ron G. Rosenfeld,and Youngman Oh
   (ii) TITLE OF INVENTION: MODULATION OF NEUROENDOCRINE DIFFERENTIATION BY PROTEIN 25.1
   (iii) NUMBER OF SEQUENCES:
   (iv) CORRESPONDENCE ADDRESS:
      (A) ADDRESSEE: DAVIS WRIGHT TREMAINE
      (B) STREET: 1501 Fourth Avenue, 2600 Century Square
      (C) CITY: Seattle
      (D) STATE: Washington
      (E) COUNTRY: U.S.A.
      (F) ZIP: 98101
   (v) COMPUTER READABLE FORM:
      (A) MEDIUM TYPE: Floppy disk
      (B) COMPUTER: PC compatible
      (C) OPERATING SYSTEM: Windows95
      (D) SOFTWARE: Word
   (vi) CURRENT APPLICATION DATA:
      (A) APPLICATION NUMBER: to be assigned
      (B) FILING DATE: 29 October 1999 (US priority document)
      (C) CLASSIFICATION:
   (viii) ATTORNEY/AGENT INFORMATION:
      (A) NAME: Davison, Barry L.
      (B) REGISTRATION NUMBER: P-47,309
      (C) REFERENCE/DOCKET NUMBER: 49321-3
   (ix) TELECOMMUNICATION INFORMATION:
      (A) TELEPHONE: 206 628 7621
      (B) TELEFAX: 206 628 7699
(2) INFORMATION FOR SEQ ID NO:1:
   (i) SEQUENCE CHARACTERISTICS:
      (A) LENGTH: 913 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 22 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 19 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:3:
   GGTCTTTCCA AACAGCCCC 19
(2) INFORMATION FOR SEQ ID NO: 4:
   (i) SEQUENCE CHARACTERISTICS:
      (A) LENGTH: 18 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:4:
   GGTCTTTCCA AACAGCCC 18
(2) INFORMATION FOR SEQ ID NO:5:
   (i) SEQUENCE CHARACTERISTICS:
      (A) LENGTH: 20 nucleotides
      (B) TYPE: nucleic acid
      (C) STRANDEDNESS: single
      (D) TOPOLOGY:, linear
   (ii) MOLECULE TYPE: oligonucleotide
   (xi) SEQUENCE DESCRIPTION: SEQ ID NO:5:
   CTCCTGGGTC TTTCCAAACA 20
(2) INFORMATION FOR SEQ ID NO:6:
   (i) SEQUENCE CHARACTERISTICS:
      (A) LENGTH: 15 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:6:
   TCCAAACAGC CCCAT 15
(2) INFORMATION FOR SEQ ID NO:7:
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:7:
   TCTTTCCAAA CAGCCCCATG 20
(2) INFORMATION FOR SEQ ID NO:8:
   (i) SEQUENCE CHARACTERISTICS:
      (A) LENGTH: 18 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:8:
   CTTTCCAAAC AGCCCCAT 18
(2) INFORMATION FOR SEQ ID NO:9:
   (i) SEQUENCE CHARACTERISTICS:
      (A) LENGTH: 17 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:9:
   CCATGACGAA CTGAACC 17
(2) INFORMATION FOR SEQ ID NO:10:
   (i) SEQUENCE CHARACTERISTICS:
      (A) LENGTH: 15 nucleotides
      (B)- TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:10:
   CAGCCCCATG ACGAA 15
(2) INFORMATION FOR SEQ ID NO:11:
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:11:
   GCCCCATGAC GAACTGAACC 20
(2) INFORMATION FOR SEQ ID NO:12:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:12:
   CCCCATGACG AACTGAACC 19
(2) INFORMATION FOR SEQ ID NO:13:
   (i) SEQUENCE CHARACTERISTICS:
      (A) LENGTH: 20 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:13:
   TTCCAAACAG CCCCATGACG 20
(2) INFORMATION FOR SEQ ID NO:14:
   (i) SEQUENCE CHARACTERISTICS:
      (A) LENGTH: 18 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:14:
   TCCAAACATC CCCATGAC 18
(2) INFORMATION FOR SEQ ID NO:15:
   (i) SEQUENCE CHARACTERISTICS:
      (A) LENGTH: 17 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:15:
   CAGCCCCATG ACGAACT 17
(2) INFORMATION FOR SEQ ID NO:16:
   (i) SEQUENCE CHARACTERISTICS:
      (A) LENGTH: 21 nucleotides
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:16:
   ACAGCCCCAT GACGAACTGA A 21